(19) Europäisches Patentamt
European Patent Office
Office européen des brevets

(11) Numéro de publication: **0 487 620 B1**

# (12) FASCICULE DE BREVET EUROPEEN

(45) Date de publication de fascicule du brevet: **19.10.94**

(51) Int. Cl.[5]: **C07C 323/60**, C07C 323/64, C07C 323/65, C07D 295/32, A61K 31/22, A61K 31/535

(21) Numéro de dépôt: **90913213.6**

(22) Date de dépôt: **23.08.90**

(86) Numéro de dépôt internationale : **PCT/FR90/00627**

(87) Numéro de publication internationale : **WO 91/02718 (07.03.91 91/06)**

(54) **NOUVEAUX DERIVES D'AMINO-ACIDES, LEUR PROCEDE DE PREPARATION ET LEUR APPLICATION THERAPEUTIOUE.**

(30) Priorité: **24.08.89 FR 8911224**

(43) Date de publication de la demande: **03.06.92 Bulletin 92/23**

(45) Mention de la délivrance du brevet: **19.10.94 Bulletin 94/42**

(84) Etats contractants désignés: **AT BE CH DE DK ES FR GB IT LI LU NL SE**

(56) Documents cités:
**EP-A- 0 072 868**
**EP-A- 0 254 032**

**Biochemical and Biophysical Research Communications, volume 116, no. 1, 14 octobre 1983, Academic Press, Inc., Duluth, Minn., US; W.W.-C. CHAN:"L-leucinthiol - a potent inhibitor of leucine aminopeptidase", see pages 297-302**

(73) Titulaire: **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur: **ROOUES, Bernard**
**12, rue Eugène-Delacroix**
**F-94410 S.-Maurice (FR)**
Inventeur: **FOURNIE-ZALUSKI, Marie-Claude**
**16, avenue de Bouvines**
**F-75011 Paris (FR)**

(74) Mandataire: **Phélip, Bruno et al**
**c/o Cabinet Harlé & Phélip**
**21, rue de La Rochefoucauld**
**F-75009 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 487 620 B1

## Description

L'invention a pour objet de nouveaux dérivés d'amino acides, leur procédé de préparation et leur application thérapeutique.

On sait que les peptides opioïdes naturels, ou enképhalines, sont essentiellement dégradés par deux enzymes appartenant à la classe des métallopeptidases, l'endopeptidase neutre EC 3.4 24.11 (enképhalinase) qui coupe la liaison Gly-Phe[4] (Malefroy et al., Nature, 276, 523, (1978)) et principalement l'aminopeptidase N EC 3.4 11.2 qui coupe la liaison Tyr[1]-Gly[2] (Waksman et al. Eur. J. Pharm., 117, 233 (1985)).

On connait des dérivés de dipeptides ayant une activité sur l'enképhalinase (Roques et al.), Nature, 288 286 (1980), Brevet français 80.08601) ainsi que des composés inhibant l'aminopeptidase N(Chan., Biochem. Biophys. Res. Commun. 116 (1983) 297; Chan et al., J.Biol.Chem. 257 (1982) 7955). De même des inhibiteurs mixtes des différentes activités enképhalinasiques ont été décrits (Fournié-Zaluski) et al. J. Med. Chem. 28 (1989) 1158; Brevet français 86.13413). Par rapport aux molécules inhibant sélectivement l'endopeptidase neutre ou l'aminopeptidase, ces inhibiteurs mixtes présentent des propriétés pharmacologiques plus puissantes. Cependant, ces composés qui possèdent une fonction hydroxamate, franchissent très mal la barrière hématoencéphalique et ne présentent donc des propriétés analgésiques importantes qu'après injection intracérébroventriculaire (i.c.v.).

L'un des objets de l'invention est de fournir de nouveaux composés capables d'inhiber conjointement les deux activités enzymatiques responsables de la dégradation des enképhalines et de manifester leurs propriétés pharmacologiques après injection intraveineuse (i.v.) sous cutanée (s.c.) ou par voie orale (per os).

Un autre objet de l'invention est de fournir de nouveaux composés, dérivés d'amino-acides, qui présentent des propriétés des substances morphiniques en particulier l'analgésie, les effets bénéfiques sur le comportement (sédation, euphorie, récompense), et les effets périphériques (antidiarréique, antitusif, hypotenseur, antiinflammatoire...) sans en avoir les inconvénients majeurs (tolérance, dépendance...).

Les nouveaux composés selon la présente invention ne possèdent pas de fonction hydroxamate mais sont caractérisés par une liaison disulfure.

D'autre part, les nouveaux composés sont susceptibles d'inhiber conjointement les deux enzymes de dégradation des enképhalines endogènes ou de potentialiser l'action d'analogues administrés de manière exogène.

L'invention a plus particulièrement pour objet des composés répondant à la formule (I).

$$H_2N\text{-}CH(R_1)\text{-}CH(R_2)\text{-}S\text{-}S\text{-}CH(R_3)\text{-}CH(R_4)\text{-}A\text{-}B\text{-}Z \qquad (I)$$

dans laquelle :

$R_1$ représente :

- une chaîne hydrocarbonée saturée ou insaturée linéaire ou ramifiée comportant de 1 à 6 atomes de carbone, substituée ou non par un groupe OR dans laquelle R est un hydrogène, une chaîne hydrocarbonée linéaire ou ramifiée de C1 à C4, ou un reste phényle (Phe) ou benzyle ; un groupe thioéther SR, R ayant la même définition que ci-dessus ou un groupe thioéther oxydé S(O)R, R ayant la même définition que ci-dessus,
- un groupe méthyl cycloalkyle à 5 à 6 atomes de carbone, un groupe benzyl ou phényl, éventuellement substitué :
  . par 1 à 5 atomes d'halogène notamment de fluor,
  . ou par un groupe OR' ou SR', R' étant un groupement alkylé de 1 à 4 atomes de carbone, le thioéther SR' étant oxydé ou non sur l'atome de soufre,
  . ou par un groupe amino éventuellement mono- ou disubstitué par un groupe alkyle aliphatique de 1 à 6 atomes de carbone, oxydé ou non sur la fonction amine,
- un groupe méthylène substitué par un hétérocycle à 5 ou 6 atomes de carbone, aromatique ou saturé, possédant comme hétéroatome, un atome d'azote, un atome d'oxygène ou un atome de soufre, les atomes d'azote et de soufre étant éventuellement oxydés sous forme de N-oxyde ou de S-oxyde.

$R_2$ représente indépendamment un atome d'hydrogène ou un groupement méthyle.

$R_3$ représente un atome d'hydrogène ou une chaîne alkyle linéaire ou ramifiée.

$R_4$ représente :

- une chaîne hydrocarbonée saturée ou insaturée, linéaire ou ramifiée comportant de 1 à 6 atomes de carbone, substitué ou non par un groupe hydroxy, éther OR dans lequel le groupe R a la même signification que précédemment, un groupe thiol SH, thioether SR (R ayant la même signification que

précédemment) ou un groupe thioether oxydé en sulfoxyde,

- un groupe méthylcycloalkyle de 5 à 6 atomes de carbone, un groupe benzyl ou phényl, éventuellement substitué par 1 à 5 atomes d'halogène notamment de fluor, par un hydroxyle ou un thiol, un éther ou un thioether OR' ou SR', R' ayant de 1 à 4 atomes de carbone, le thioether étant oxydé ou non sur l'atome de soufre, par un groupe amino éventuellement mono ou disubstitué par un groupe alkylealiphatique de 1 à 6 atomes de carbone, oxydé ou non sur la fonction amine,
- un groupe méthylène substitué par un hétérocycle à 5 ou 6 atomes de carbone, aromatique ou saturé, possédant comme hétéroatome, un atome d'azote, un atome d'oxygène ou un atome de soufre, les atomes d'azote et de soufre étant éventuellement oxydés sous forme de N-oxyde ou de S-oxyde.

Z peut représenter:

* un groupement $-CH(R_5)-[CH(R_6)]_n-COO(R_7)$

dans lequel

$R_5$ et $R_6$ peuvent représenter indépendamment l'un de l'autre :

. un hydrogène,

. un groupement alkyle linéaire ou ramifié de 1 à 6 atomes de carbone substitué ou non par des groupements hydroxy ou éther OR, thiol SH ou thioether, SR (R ayant la définition précisée précédemment),

. un groupement phényl (Phe) ou benzyl substitué ou non par 1 à 5 atomes d'halogène ; un groupement OR' ou SR', R' ayant la définition précisée ci-dessus.

$R_5$ et $R_6$ peuvent également représenter chacun des chaînes hydrocarbonées saturées ou insaturées contenant de 1 à 6 atomes de carbone dans lesquels 1 à 2 atomes de carbone peuvent être substitués par un atome d'oxygène, de soufre ou d'azote pouvant former un ou plusieurs cycles d'hydrocarbones saturés ou aromatiques de 5 à 6 maillons ou d'hétérocycles saturés ou aromatiques de 5 à 6 maillons.

n peut être égal à 0 ou à 1.

$R_7$ représente :

. un hydrogène,

. ou un groupement hydrocarboné linéaire ou ramifié de 1 à 6 atomes de carbone saturé ou insaturé,

. ou un groupement benzyle.

* un groupement morpholinyle $-N-C_4H_8O$
* un groupement piperidinyle $-N-C_5H_{10}$, éventuellement substitué par un reste -OH, un reste $-C0_2H$, un reste $COOR^1$, dans lequel $R^1$ représente une chaîne alkyle linéaire ou ramifiée contenant 1 à 6 atomes de carbone, un reste phényle ou un reste benzyle.
* un groupement pyralozinyle-$N-C_4H_8-NH$, un groupement pyralozinyle substitué $-NC_4H_8-N-R^2$ dans lequel $R^2$ représente une chaîne alkylée contenant de 1 à 6 atomes de carbone, un reste benzyle ou un reste phényle.

A-B représente un groupement amide CONH ou rétro amide NHCO.

Les composés de formule (I) ont de 2 à 6 atomes de carbones asymétriques. Ils existent donc sous forme de mélanges racémiques, sous formes d'énantiomètres, de diastéréoisomères ou de stéréoisomères. L'invention a également pour objet les sels d'addition des composés de formule (I), obtenus avec des acides organiques ou minéraux pharmacologiquement acceptables tels que le chlorhydrate, le bromhydrate, les sulfates, les nitrates, les borates, les phosphates, le méthane sulfonate, l'acétate, le fumarate, le succinate, l'ascorbate, l'oxalate, le lactate, le pyruvate, le citrate, le tartrate, le maléate, le malonate, le benzoate, le salicylate, le dichloro-2,6 benzoate, le triméthoxy benzoate, le diaminobenzène sulfonate, le chromoglycate, le benzène sulfonate, le cyclohexane sulfonate, le toluène sulfonate, le dipropyl acétate, le glucose-1 phosphate, le pamoate et le palmitate.

De manière préférentielle, les composés selon l'invention sont tels que dans la formule (I) si Z représente un groupement $-CH(R_5)-[CH(R_6)]_n-COO-(R_7)$.

$R_2$ et $R_3$ représentent des atomes d'hydrogène,

AB représente une liaison amide CONH,

$R_1$ représente un groupement $- CH_2 CH_2 S(O) CH_3$ et

$R_7$ représente un groupement $- CH_2Phe$, auquel cas ils peuvent répondre aux formules :

$H_2N-CH(CH_2CH_2S(O)CH_3)-CH_2S-S-CH_2-CH(CH_2PHe)CONHCH_2COOCH_2Phe$

ou

$H_2N-CH(CH_2CH_2S(O)CH_3)-CH_2-S-S-CH_2-CH-(CH_2Phe)CONH-CH(CH_2Phe)COOCH_2Phe$

ou

$H_2NCH(CH_2CH_2S(O)CH_3)-CH_2-S-S-CH_2-CH(CH_2PHe)CONH-CH-CH-$
$COOCH_2Phe$

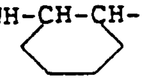

et si $R_4$ représente un groupement $-CH_2CH_2S(0)CH_3$, ils peuvent répondre aux formules :

$H_2N-CH(CH_2CH_2S(O)CH_3)-CH_2S-S-CH_2-CH(CH_2CH_2S(O)CH_3)$
$CONHCH(CH_3)-COOCH_2Phe$

ou

$H_2N-CH(CH_2CH_2S(O)CH_3)-CH_2-S-S-CH_2-CH(CH_2CH_2S(O)CH_3)CONH-$
$CH(CH_2Phe)CH_2COOCH_2Phe$

ou

$H_2N-CH(CH_2CH_2S(O)CH_3)-CH_2-S-S-CH_2-CH(CH_2CH_2S(O)CH_3)CONH-$
$CH(Phe)CH_2COOCH_2Phe$

ou

$H_2N-CH(CH_2CH_2S(O)CH_3)-CH_2-S-S-CH_2-CH(CH_2CH_2S(O)CH_3)CONH-$
$CH-CH-COOCH_2Phe$

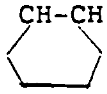

Les composés selon l'invention peuvent aussi répondre de manière préférentielle aux formules :

$H_2NCH(CH_2CH_2SCH_3)CH_2-S-S-CH_2CH(CH_2CH(CH_3)_2)CONHCH(CH_3)COOCH_2Phe$

ou

$H_2N-CH(CH_2CH_2SCH_3)CH_2-S-S-CH_2-CH(CH_2Phe)CONHCH(CH_3)COOCH_2Phe$

ou

$H_2NCH(CH_2CH_2SCH_3)CH_2-S-S-CH_2CH(CH_2CH_2SCH_3)CONHCH(CH_3)COOCH_2Phe$

ou

$H_2N-CH(CH_2CH_2SCH_3)CH_2-S-S-CH_2-CH(CH_2Phe)CONHCH(CH_2Phe)COOCH_2Phe$

ou

$H_2N-CH(CH_2CH(CH_3)_2)-CH_2-S-S-CH_2-CH(CH_2Phe)CONH-CH_2COOCH_2Phe$

ou

$H_2N-CH(CH_2CH(CH_3)_2)-CH_2-S-S-CH_2-CH(CH_2Phe)CONH(CH_2Phe)COOCH_2Phe$

ou

$H_2N-CH(CH_2CH_2SCH_3)-CH_2-S-S-CH_2-CH(CH_2Phe)CONH-CH_2-COOCH_2Phe$

ou

$H_2N-CH(CH_2CH(CH_3)_2)-CH_2-S-S-CH_2-CH(CH_2Phe)CONH-CH(CH_3)COOCH_2Phe$

ou

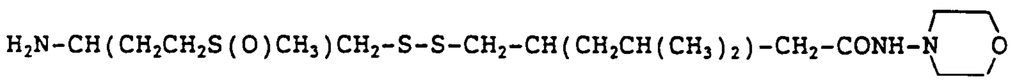

Ces derniers composés sont particulièrement interessants du fait de leurs propriétés de biodisponibilité (actif per os).

L'invention a d'autre part pour objet un procédé de préparation des composés de formule (I) précédemment définis dans laquelle Z représente un groupement

$-CH(R_5)-[CH(R_6)]_n-CO(R_7)$

comprenant les étapes suivantes :

- des composés de formule (II) :

  $R_8NH-CH(R_1)-CH(R_2)SH$

  dans laquelle $R_8$ représente un groupement protecteur de la fonction amine sont condensés sur la dithiodipyridine (III)

  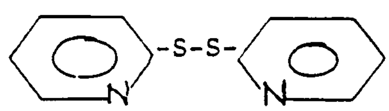

  pour conduire aux composés de formule générale (IV) :

  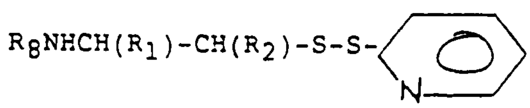

  (IV)

- puis le composé (IV) est condensé sur les composés de formule (V) :

  $HS-CH(R_3)-CH(R_4)-A-B-CH(R_5)-[CH(R_6)]_n-COO(R_7)$ (V)

  pour conduire aux composés de formule (VI) :

  $R_8NH-CH(R_1)-CH(R_2)-S-S-CH(R_3)-CH(R_4)-A-B-CH(R_5)-[CH(R_6)]_n-COO(R_7)$

- puis la fonction amine du composé (VI) est déprotégée pour conduire au composé de formule (I).

De manière préférentielle, la condensation du composé de formule (II) sur la dithiopyridine (III) s'effectue à température ambiante en présence d'acide acétique dans l'éthanol.

La condensation du composé intermédiaire (IV) sur le composé (V) est effectuée dans l'éthanol à température ambiante.

Les produits intermédiaires (II) et (V) sont synthétisés en suivant les modes opératoires décrits dans la littérature (Chan, Biochem. Biophys. Res. Commun., 116, 297, (1983) et Cushman et al., Biochemistry, 16, 5484, (1977)).

Le groupe protecteur $R_8$ est préférentiellement le t-Butylocarboxyl (t.Boc). Ce groupe peut être éliminé à l'aide d'acide trifluoroacétique dans du chlorure de méthylène.

Si $R_2$ représente un atome d'hydrogène, le produit de formule (II) peut être obtenu à partir d'un bêta-amino alcool de formule :

$H_2N$-$CH$-$(R_1)$-$CH_2OH$ (VII)

- par protection de la fonction amine à l'aide d'un groupe $R_8$, résultant en la formation du produit de formule :

  $R_8NH$-$CH(R_1)$ - $CH_2OH$ (VIII)

- puis par activation de la fonction alcool, notamment sous la forme de tosylate, résultant en la formation d'un produit de formule :

  $R_8HN$-$CH(R_1)$ $CH_2OT_S$ (IX)

  dans laquelle $T_S$ représente un groupement tosylate,
- puis par transformation du groupement tosylate en groupement acétylthio résultant en la formation du produit de formule :

  $R_8NH$ - $CH(R_1)$-$CH_2$-S-$COCH_3$ (X)

- puis par hydrolyse alcaline en atmosphère réductrice du produit (X) résultant en la formation du composé de formule (II).

Les produits béta-amino alcools de formule (VII) sont obtenus par un protocole décrit dans la littérature [(Chan. Biochem. Biophys. Res. Commun. 116 297 (1983)].

Si $R_2$ représente un atome d'hydrogène, le produit de formule (X) peut être obtenu à partir du produit de formule (VIII), dans les conditions de la réaction de Mitsunobu, (Synthesis (1981), 1-28 c'est à dire en présence de triphényl phosphine et d'un azodicarboxylate de dialkyle.

Si $R_2$ représente un groupe -$CH_3$ et si $R_1$ et $R_4$ ont la même signification, le composé de formule (II) peut être préparé par réarrangement selon Curtius (Pataï, The Chemistry of Azids groups, 397, (1971)), à partir d'un composé de formule :

$CH_3$-CO-$SCH(CH_3)CHR_1$-$CO_2H$ (XI)

- par traitement dudit composé à l'azoture de sodium $NaN_3$ en présence de dicyclohexylcarbodiimide (DCC) résultant en la formation du produit de formule :

  $CH_3$-CO-$SCH(CH_3)CHR_1$-$CON_3$ (XII)

- puis par chauffage en présence d'un alcool résultant en la formation du produit de formule :

  $CH_3$-CO-$SCH(CH_3)CHR_1$-$NHR_8$ (XIII)

- puis par hydrolyse acide du groupement acétylthio, dans des conditions réductrices.

De manière préférentielle, la fonction amine du composé (XIII) est protégée sous forme de carbamate.

Si $R_3$ représente un atome d'hydrogène ou un groupe -$CH_3$, et AB un groupement amide CONH, le composé de formule (V) peut être obtenu à partir d'un composé de formule :

$CH_3$-CO S $CH(R_3)CH(R_4)$-$CO_2H$ (XIV)

qui sont des acides acétylthyo-propanoïques substituées en 2, (Cushman et al. (1977), Biochemistry 16 5484).

- par hydrolyse alcaline préférentiellement dans des conditions oxydantes dudit composé, résultant en la formation du composé de formule :

  $(SCH(R_3)CH(R_4)$-$CO_2H)_2$ (XV)

- puis condensation dans les conditions classiques de couplage peptidique avec un amino-ester de formule :

$H_2N\text{-}CH(R_5)\text{-}[CH(R_6)]_n\text{-}CO\text{-}OR_7$ (XVI)

résultant en la formation d'un composé de formule :

$(S\ CH(R_3)CH(R_4)CONH\ CH(R_5)[CH(R_6)_n]\text{-}COOR_7)_2$ (XVII)

- puis traitement en milieu réducteur (zinc et acide chlorydrique).

Les béta-amino acides de formule (XVI) sont synthétisés par des méthodes classiques (Xie et col., J. Med. Chem. (1989), 32, 1497).

Le composé de formule XI peut être obtenu à partir de l'ylure de phosphore de formule :

$(Phe)_3P\text{-}CH(R_1)\text{-}COO\text{-}CH_3$ (XVIII)

- par condensation de Wittig avec de l'acétaldéhyde résultant en la formation du composé de formule :

$CH_3CH = C(R_1)\text{-}CO\text{-}CH_3$ (XIX)

- puis par hydrolyse alcaline résultant en la formation du composé de formule :

$CH_3CH = C(R_1)CO_2H$ (XX)

- puis par addition d'acide thioacétique.

Si $R_3$ représente un atome d'hydrogène ou un groupe $-CH_3$ et si $R_1$ et $R_4$ ont la même signification et si AB représente un groupe rétro-amide NHCO, le composé de formule (V) peut être préparé respectivement à partir des composés (X) et (XIII) répondant à la formule générale :

$CH_3CO\text{-}S\text{-}CH(R_2)CH(R_1)NHR_8$ (XXI)

- par hydrolyse alcaline dans des conditions oxydantes résultant en la formation du composé de formule :

$[SCH(R_2)CH(R_1)NHR_8]_2$ (XXII)

- puis par déprotection de la fonction amine conduisant au composé de formule :

$[SCH(R_2)CH(R_1)NH_2]_2$ (XXIII)

- puis par condensation dans les conditions classiques de couplage peptidique avec des monoacides esters maloniques ou succiniques de formule :

$CO_2H\text{-}CH(R_5)\text{-}[CH(R_6)]_n\text{-}COOR_7$ (XXIV)

résultant en la formation d'un composé de formule :

$[SCH(R_2)CHR_1\text{-}NHCO\text{-}CHR_5\text{-}(CHR_6)_n\text{-}COOR_7]_2$

- puis par traitement en milieu réducteur.

Les monoacides monoesters maloniques ou succiniques de formule (XXIV) sont synthétisés par des méthodes classiques (Fournie-Zaluski et coll., Int. J. Pept. Prot. Res., 33, (1989), 146).

L'invention a de plus pour objet un procédé de préparation des composés de formule I dans laquelle le groupement Z représente :

* un groupement morpholinyle $-N\text{-}C_4H_8O$
* un groupement piperidinyle $-N\ C_5H_{10}$, éventuellement substitué par un reste -OH, un reste $-CO_2H$, un reste $COOR^1$, dans lequel $R^1$ représente une chaîne alkyle linéaire ou ramifiée contenant 1 à 6

atomes de carbone, un reste phényle ou un reste benzyle,

un groupement pyralozinyle-N-$C_4H_8$-NH, un groupement pyralozinyle substitué -$NC_4H_8$-N-$R^2$ dans lequel $R^2$ représente une chaîne alkylée contenant de 1 à 6 atomes de carbone, un reste benzyle ou un reste phényle,

caractérisé en ce que des composés de formule

$(SCH(R_3)CH(R_4)\text{-}CO_2H)_2$ (XV)

sont condensés avec une hydrazine de formule

$H_2N\text{-}Z$ (XXV)

résultant en la formation d'un composé de formule

$(SCH(R_3)CH(R_4)\text{-}CONH\text{-}Z)_2$ (XXVI)

qui est condensé sur un composé de formule

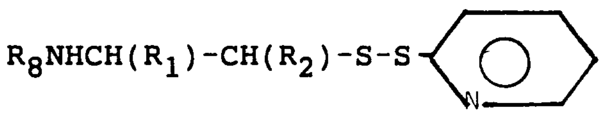

(IV)

pour conduire au composé de formule (I).

Un autre objet de l'invention est une composition pharmaceutique contenant au moins un composé tel que défini précédemment, ou obtenu par le procédé tel que défini précédemment en association avec un ou plusieurs diluants ou adjuvants compatibles ou pharmaceutiquement acceptables.

Ces compositions peuvent être utilisées en particulier comme analgésique majeur, comme antalgique, et comme antidépresseur. Ces composés sont pratiquement dénués des effets secondaires des morphiniques (tolérance et dépendance). Leur principale application est donc dans le domaine de l'analgésie et des antidépresseurs.

Un autre objet de l'invention est l'utilisation des composés tels que définis précédemment ou obtenus par un procédé tel que défini précédemment pour la fabrication de médicaments pour le traitement de la douleur ou de la dépression.

L'invention sera encore illustrée sans être aucunement limitée par les exemples ci-après:

La liste des composés préparés selon les exemples 1 à 32 est donnée dans le Tableau I.

Pour tous les composés décrits dans ce tableau, $R_2$ et $R_3$ représentent tous deux des atomes d'hydrogène, $R_7$ représente un groupement $-CH_2Phe$ et AB une liaison amide CONH.

TABLEAU I

| Exemple | $R_1$ | $R_4$ | $R_5$ | $R_6$ |
|---|---|---|---|---|
| 1 | $CH_2CH(CH_3)_2$ | $CH_2Phe$ | H | - |
| 2 | $CH_2CH(CH_3)_2$ | $CH_2Phe$ | $CH_3$ | - |
| 3 | $CH_2CH(CH_3)_2$ | $CH_2Phe$ | $CH_2Phe$ | - |
| 4 | $CH_2Phe$ | $CH_2Phe$ | H | - |
| 5 | $CH_2CH_2SCH_3$ | $CH_2Phe$ | H | - |
| 6 | $CH_2CH_2SCH_3$ | $CH_2Phe$ | $CH_3$ | - |
| 7 | $CH_2CH_2SCH_3$ | $CH_2Phe$ | $CH_2Phe$ | - |
| 8 | $CH_2CH_2S(O)CH_3$ | $CH_2Phe$ | H | - |
| 9 | $CH_2SCH_3$ | $CH_2Phe$ | H | - |
| 10 | $CH_2S(O)CH_3$ | $CH_2Phe$ | H | - |
| 11 | $CH_2SCH_2Phe$ | $CH_2Phe$ | H | - |
| 12 | $CH_2S(O)CH_2Phe$ | $CH_2Phe$ | H | - |
| 13 | $CH_2CH_2SCH_3$ | $CH_2CH(CH_3)_2$ | $CH_3$ | - |
| 14 | $CH_2CH_2S(O)CH_3$ | $CH_2CH(CH_3)_2$ | $CH_3$ | - |
| 15 | $CH_2SCH_3$ | $CH_2CH(CH_3)_2$ | $CH_3$ | - |
| 16 | $CH_2S(O)CH_3$ | $CH_2CH(CH_3)_2$ | $CH_3$ | - |
| 17 | $CH_2SCH_2Phe$ | $CH_2CH(CH_3)_2$ | $CH_3$ | - |
| 18 | $CH_2S(O)CH_2Phe$ | $CH_2CH(CH_3)_2$ | $CH_3$ | - |
| 19 | $CH_2StBu$ | $CH_2CH(CH_3)_2$ | $CH_3$ | - |
| 20 | $CH_2S(O)tBu$ | $CH_2CH(CH_3)_2$ | $CH_3$ | - |
| 21 | $CH_2CH_2SCH_3$ | $CH_2CH_2SCH_3$ | $CH_3$ | - |
| 22 | $CH_2CH_2S(O)CH_3$ | $CH_2CH_2S(O)CH_3$ | $CH_3$ | - |
| 23 | $CH_2CH_2SCH_3$ | $CH_2Phe$ | Phe | H |
| 24 | $CH_2CH_2S(O)CH_3$ | $CH_2Phe$ | Phe | H |
| 25 | $CH_2CH_2SCH_3$ | $CH_2Phe$ | $CH_2-CH_2-CH_2-CH_2$ | |
| 26 | $CH_2CH_2S(O)CH_3$ | $CH_2Phe$ | $CH_2-CH_2-CH_2-CH_2$ | |
| 27 | $CH_2CH_2SCH_3$ | $CH_2CH_2SCH_3$ | Phe | H |
| 28 | $CH_2CH_2S(O)CH_3$ | $CH_2CH_2S(O)CH_3$ | Phe | H |
| 29 | $CH_2CH_2SCH_3$ | $CH_2CH_2SCH_3$ | $CH_2Phe$ | H |
| 30 | $CH_2CH_2S(O)CH_3$ | $CH_2CH_2S(O)CH_3$ | $CH_2Phe$ | H |
| 31 | $CH_2CH_2SCH_3$ | $CH_2CH_2SCH_3$ | $CH_2-CH_2-CH_2-CH_2$ | |
| 32 | $CH_2CH_2S(O)CH_3$ | $CH_2CH_2S(O)CH_3$ | $CH_2-CH_2-CH_2-CH_2$ | |

Exemple 1 Préparation de $H_2N-CH(CH_2CH(CH_3)_2)-CH_2-S-S-CH_2-CH(CH_2Phe)CONH-CH_2COOCH_2Phe$

Etape 1 Préparation de $Boc-NH-CH[CH_2-CH(CH_3)_2]CH_2-S-S-$ 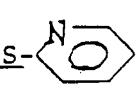

A une solution de 3 g de 2,2' dithiodip dans 5 ml d'éthanol dégazé et 0,35 ml d'acide acétique, on ajoute une solution de 1,6 g de $Boc-NH-CH[(CH_2-CH(CH_3)_2]-CH_2-SH$ dans 5 ml d'éthanol dégazé, on agite 24h à température ambiante. On évapore à sec. On obtient une huile jaune que l'on chromatographie sur gel de silice dans un mélange cyclohéxane/acétate d'éthyle/acide acétique : 9/1,25/0,25. On obtient 1,62 g du produit attendu. Rdt 72 % ; Rf (cyclohéxane/acétate d'éthyle = 8/2) = 0,25

Etape 2 Préparation de $Boc-NH-CH-[CH_2CH(CH_3)_2]CH_2-S-S-CH_2-CH(CH_2Phe)-CONH-CH_2-COO-CH_2Phe$

A une solution de 1,6 g du composé précédent dans 10 ml d'éthanol dégazé, on ajoute une solution de 1,7 g de N(mercaptométhyl-2-phényl-3-oxo-1-propylglycine benzyl ester dans 10 ml d'éthanol dégazé. On agite 24h à température ambiante. On évapore à sec. On obtient une huile jaune, chromatographiée sur gel de silice avec un mélange cyclohexane /EtOAc/AcOH = 7/3/0,5 comme éluant.

On obtient 2,42 g (Rdt 86 %) du produit attendu. Rf (cyclohéxane/acétate d'éthyle/acide acétique = 8/2/0,5) = 0,38.

Etape 3 Préparation du produit final

On dissout 2,4 g du composé précédent dans 4 ml de $CH_2Cl_2$. On ajoute à 0°C 6 ml d'acide trifluoroacétique (TFA). On agite 2 h à 0°C, et 2 h à température ambiante. On évapore à sec. On chromatographie sur gel de silice en utilisant le mélange $CH_2Cl_2$/MeOH = 20/1 comme éluant. On obtient 1,63 g du produit attendu (Rdt 80 %). Rf ($CH_2Cl_2$/MeOH = 9/1) = 0,46.

Exemple 2

Préparation de $H_2N-CH(CH_2CH(CH_3)_2)CH_2-S-S-CH_2-CH(CH_2Phe)CONH-CH(CH_3)COOCH_2Phe$

A partir de 0,685 g de boc amino-2 méthyl-4 pentane-1 dithio-1'-piperidine préparé dans l'exemple 1, étape 1, et 0,715 g de N[mercapto méthyl-2 phényl-3 oxo-1 propane]-alanine benzyl ester, on obtient selon les conditions de l'exemple 1, étape 2, l'inhibiteur protégé (0,88 g, 75 %). Après déprotection, on obtient le composé attendu (0,57 g, 80 %), sous forme d'un produit huileux, Rf (cyclohexane/EtoAc/AcOH = 8/2/0,5) = 0,39. Spectre de masse IC, $NH_3$ (M+1) = 489.

Exemple 3

Préparation de $H_2N-CH(CH_2CH(CH_3)_2)CH_2-S-S-CH_2-CH(CH_2Phe)CONH-CH(CH_2Phe)COO-CH_2Phe$.

A partir de 0,685 g de boc amino-2 méthyl-4 pentane-1 dithio-1'-pyridine préparé dans l'exemple 1, étape 1, et 0,870 g de N[mercapto méthyl-2 phényl-3 oxo-1 propane]-phénylalanine benzyl ester, selon les conditions de l'exemple 1, étape 2, on obtient l'inhibiteur protégé (0,90 g, 60 %). Après déprotection, on obtient le composé attendu (0,554 g, 72 %), Rf (cyclohexane/EtoAc/AcOH = 8/2/0,5) = 0,41. Spectre de masse IC, $NH_3$ (M+1) = 565.

Exemple 4

Préparation de $H_2N-CH(CH_2Phe)CH_2-S-S-CH_2-CH(CH_2Phe)-CONH-CH_2-COOCH_2Phe$

**Etape 1 Préparation de t.Boc-NH-CH($CH_2$Phe)-$CH_2$-S-S-** 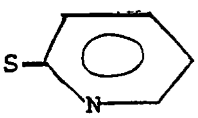

A partir de 410 mg de dipyridine-2-2'-disulfure et 250 mg de t-Boc-amino-2 phényl-3-propane thiol-1, on obtient en suivant les conditions opératoires de l'exemple 1, 170 mg (Rdt 42 %) du produit attendu sous forme d'une huile. Rf (cyclohexane/EtoAc/AcOH = 9/10/0,5) = 0,26.

Etape 2 Préparation de $t-Boc-NH-CH(CH_2Phe)-CH_2S-S-CH_2CH(CH_2Phe)-CONH-COO-CH_2Phe$

A partir de 170 mg du composé précédent et 160 mg de N(mercapto-méthyl-2-phényl-3-oxo-1-propane)-glycine benzyl ester, on obtient en suivant les conditions expérimentales de l'exemple 1, 174 mg (61 %) du produit attendu sous forme d'une huile : Rf (cyclohexane/EtoAc/AcOH = 7/3/0,5) = 0,54.

Etape 3 Préparation du produit final

A partir de 170 mg du composé précédent, on obtient en suivant les conditions expérimentales de l'exemple 1, 80 mg (Rdt 55 %) du produit attendu sous forme huileuse. Rf ($CH_2Cl_2$/MeOH = 9/1) = 0,56.

Exemple 5

Préparation de $H_2N-CH(CH_2CH_2SCH_3)-CH_2-S-S-CH_2-CH(CH_2Phe)-CONH-CH_2COOCH_2Phe$

**Etape 1 Préparation de t.Boc-NH-CH($CH_2$-$CH_2$-S-$CH_3$)-$CH_2$-S-S-** 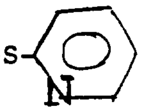

A partir de 0,82 g de t-Boc-amino-2 mercaptométhyl-4 butane thiol-1 et 1,43 g de dipyridine 2,2' disulfure, on obtient en suivant le mode opératoire de l'exemple 1, 0,77 g (Rdt 68 %) du produit attendu sous forme d'une huile. Rf (cyclohexane/Et/OAc/AcOH = 8/2/0,5) = 0,32.

Etape 2 Préparation de t-Boc-NH-CH($CH_2$-$CH_2$-S-$CH_3$)-$CH_2$-S-S-$CH_2$CH($CH_2$Phe)CONH-$CH_2$-COO-$CH_2$Phe

A partir de 0,75 g du composé précédent et 0,76 g de N(mercapto/méthyl-2 phényl-3 oxo-1-propane)-glycine benzyl ester, on obtient en suivant les conditions expérimentales de l'exemple 1, 1,18 g (Rdt 87 %) du produit attendu sous forme d'une huile. Rf (cyclohexane/EtoAc/AcOH = 0,46.

Etape 3 Préparation du produit final

A partir de 0,4g du composé précédent, on obtient en utilisant les conditions expérimentales de l'exemple 1, 0,35 g du produit attendu (Rdt 85 %) sous forme d'une huile. Rf ($CH_2Cl_2$/MeOH = 9/1) = 0,50.

Exemple 6

Préparation du $H_2N-CH(CH_2CH_2SCH_3)CH_2-S-S-CH_2-CH(CH_2Phe)CONH-CH(CH_3)COOCH_2Phe$

A partir de 0,9 g de boc amino-2 mercapto méthyl-4 butane-1 dithio-1'-pyridine préparé dans l'exemple 3, étape 1, et 0,88 g de N[mercapto méthyl-2 phényl-4 oxo-1 propane]-alanine benzyl ester, selon les conditions de l'exemple 1, étape 2, on obtient l'inhibiteur protégé (1,07 g, Rdt 70 %). Après déprotection selon le procédé de l'exemple 1, étape 3, on obtient 0,66 g (75 %) du produit attendu. Rf ($CH_3$/MeOH/AcOH = 9/1/0,5) = 0,62. Spectre de masse IC, $NH_3$ (M+1) = 507.

Exemple 7

Préparation du $H_2N-CH(CH_2CH_2SCH_3)CH_2-S-S-CH_2-CH(CH_2Phe)CONH-CH(CH_2Phe)COOCH_2Phe$.

A partir de 0,7 g de boc amino-2 mercapto méthyl-4 butane-1 dithio-1'-pyridine préparé dans l'exemple 3, étape 1, et 0,9 g de N[mercapto méthyl-2 phényl-3 oxo-1 propane]-phénylalaninebenzyl ester, selon les conditions de l'exemple 1, étape 2, on obtient l'inhibiteur protégé (0,8 g, Rdt 60 %). Après déprotection selon le procédé de l'exemple 1, étape 3, on obtient 0,616 g (72 %) du produit attendu. Rf ($CHCl_3$/MeOH/AcOH = 9/1/0,5) = 0,65 et 0,75. Spectre de masse IC, $NH_3$ (M+1) = 583.

Exemple 8

Préparation du $H_2N-CH(CH_2CH_2S(O)CH_3)-CH_2S-S-CH_2-CH(CH_2Phe)-CONH-CH_2COOCH_2Phe$

Ce composé est obtenu à partir du composé de l'exemple 5, protégé sur la fonction amine (étape 2). L'oxydation de 0,5 g de ce composé par le périodate de sodium (1 eq) en milieu hydroalcoolique permet d'obtenir 0,380 g du sulfoxyde correspondant qui est un produit huileux. Rf (cyclohexane/EtoAc/AcOH = 7/3/0,5) = 0,20 (Rdt 78 %).

Le traitement par l'acide trifluoroacétique de la même manière que dans l'étape 3 de l'exemple 1 permet de libérer le composé final attendu. P = 0,289 g (Rdt 85 %) Rf ($CH_2Cl_2$ MeOH = 9/1) = 0,25.

Exemple 9

Préparation du $H_2N-CH(CH_2SCH_3)CH_2-S-S-CH_2-CH(CH_2Phe)-CONHCH_2-COOCH_2Phe$

Etape 1 Préparation du t-boc-NHCH($CH_2SCH_3$)$CH_2$-S-S- 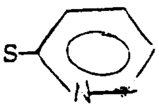

A partir de 2,2 g de N-t-Boc amino-2-mercapto-1-méthylthio-3-propane, on obtient dans les conditions de l'exemple 1, étape 1, 2,3 g du produit attendu (70 %) Rf (cyclohexane) EtOAc = 8/2) = 0,28.

Etape 2 Préparation du t-Boc $NHCH(CH_2SCH_3)CH_2-S-S-CH_2-CH$ ($CH_2Phe$) $CONHCH_2COOCH_2Phe$

A partir de 2,2 g du composé précédent, on obtient dans les conditions de l'exemple 1, étape 2, 3,07 g (82 %) du produit attendu. Rf (cyclohexane/EtOAc/AcOH = 7/3/0,5) =0,52.

Etape 3 Préparation du produit final

A partir de 3 g du composé précédent, on obtient dans les conditions de l'exemple 1, étape 3, 1,99 g du composé attendu (Rdt 78 %) - Rf ($CH_2Cl_2$/MeOH = 9/1) = 0,38.

Exemple 10

Préparation de $H_2N-CH(CH_2CH_2S(O)CH_3)-CH_2-S-S-CH_2-CH(CH_2Phe)-CONHCH_2-COOCH_2Phe$

Ce composé est obtenu à partir du composé final de l'exemple 9, protégé sur la fonction amine (étape 2). L'oxydation dans les conditions de l'exemple 9 conduit au sulfoxyde correspondant (Rdt 45 %) Rf (cyclohexane/EtoAc/ = AcOH = 7/3/0,5) = 0,30.

Le traitement par TFA (conditions de l'exemple 1, étape 3) libère le composé attendu. Rf ($CH_2Cl_2$/MeOH = 7/3) 0,35 ( Rdt 80 %).

Exemple 11

Préparation du $H_2N-CH(CH_2SCH_2Phe)CH_2S-S-CH_2CH(CH_2Phe)-CONHCH_2-COOCH_2Phe$

Etape 1 Préparation du t-Boc-NHCH($CH_2SCH_2Phe$)$CH_2$-S-S- 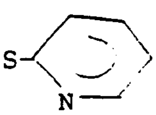

A partir de 1,48 g de N-t-Boc amino-2-mercapto-1-benzylthio-3 propane, on obtient dans les conditions de l'exemple 1, étape 18, 1,3 g (Rdt 68 %) du produit attendu. Rf (cyclohexane/Et OAc : 7/3) = 0,31.

Etape 2 Préparation de t-Boc $NH-CH(CH_2SCH_2Phe)CH_2-S-S-CH_2-CH$ ($CH_2Phe$)$CONH-CH_2-COO-CH_2Phe$

A partir de 1,38 g du composé précédent, on obtient les conditions de l'exemple 1 étape 2 1,73 g (Rdt 80 %) du composé attendu. Rf (cyclohexane/EtOAc/AcOH = 7/3/0,5) = 0,55.

Etape 3 Préparation du composé final

A partir de 1,7 g du composé précédent, on obtient, dans les conditions de l'exemple 1, étape 3, 1,04 g (70 %) du produit attendu. Rdt ($CH_2Cl_2$/MeOH = 8/2) = 0,43.

Exemple 12

Préparation du $H_2N\text{-}CH(CH_2S(O)CH_2Phe)CH_2\text{-}S\text{-}S\text{-}CH_2CH(CH_2Phe)\text{-}CONHCH_2COOCH_2Phe$

Ce composé est obtenu à partir du composé de l'exemple 11 protégé sur la fonction amine (étape 2). L'oxydation dans les conditions de l'exemple 8 conduit au sulfoxyde correspondant Rdt (70 %) Rf (cyclohexane/EtOAc/AcOH = 7/3/0,5) = 0,32

Le traitement par le TFA (exemple 1, étape 3) libère le composé attendu Rf ($CH_2Cl_2$/MeOH = 7/3) = 0,48 (Rdt 82 %).

Exemple 13

Préparation du $H_2NCH(CH_2CH_2SCH_3)CH_2S\text{-}S\text{-}CH_2CH(CH_2CH(CH_3)_2)\text{-}CONHCH(CH_3)COOCH_2Phe$

1,7 g du disulfure mixte pyridine -NBoc méthionine obtenu à l'exemple 5 étape 1, est condensé dans les conditions de l'exemple 1, étape 2, avec 1,6 g de N(mercaptométhyl-2-méthyl-4 oxo-1 pentyl) alaninate de benzyle. On obtient 1,94 g (Rdt 70 %) du composé final protégé sur la fonction amine. Rf (cyclohexane/EtOAc/AcOH = 7/3/0,5) = 0,41.

Le traitement par le TFA (exemple 1, étape 3) libère le composé final attendu (1,35 g) (Rdt 83 %) Rf ($CH_2Cl_2$/MeOH = 8/2) 0,51.

Exemple 14

Préparation du $H_2NCH(CH_2CH_2S(O)CH_3)CH_2SSCH_2CH(CH_2CH(CH_3)_2)\text{-}CONHCH(CH_3)COOCH_2Phe$

Le composé de l'exemple 13 protégé sur la fonction amine est oxydé dans les conditions décrites pour l'exemple 8. Après déprotection par le TFA (exemple 1, étape 3), on obtient l'inhibiteur attendu. Rdt global 60 %. Rf ($CH_2Cl_2$/MeOH = 8/2) = 0,36.

Exemple 15

Préparation du $H_2N\text{-}CH(CH_2SCH_3)CH_2S\text{-}S\text{-}CH_2CH\text{-}(CH_2CH(CH_3)_2)\text{-}CONH\text{-}CH(CH_3)COOCH_2Phe$

1,65 g du disulfure mixte pyridine/NBoc S-méthylcystéine thiol (exemple 9, étape 1) est condensé dans les conditions de l'exemple 1 étape 2 avec 1,6 g de N(mercapto-méthyl-2-méthyl-4 oxo-1 pentyl) alaninate de benzyle. On obtient 1,89 g (Rdt 70 %) du composé protégé sur la fonction amine. Rf (cyclohexane/EtOAc/AcOH = 7/3/0,5) = 0,45.

Le traitement par le TFA (exemple 3 étape 1) libère le composé final (1,32 g) (Rdt 82 %) Rf ($CH_2Cl_2$/MeOH : 8/2) = 0,60.

Exemple 16

Préparation du $H_2N\text{-}CH(CH_2S(O)CH_3)CH_2S\text{-}S\text{-}CH_2CH\text{-}(CH_2CH(CH_3)_2)\text{-}CONHCH(CH_3)COOCH_2Phe$

Le composé de l'exemple 15 protégé sur la fonction amine est oxydé dans les conditions décrites pour l'exemple 8. Après déprotection par le TFA (exemple 1, étape 3) on obtient le composé final attendu. Rdt global 55 %. Rf ($CH_2Cl_2$/MeOH : 8/2) = 0,38

Exemple 17

Préparation du $H_2NCH(CH_2SCH_2Phe)CH_2S\text{-}S\text{-}CH_2CH\text{-}(CH_2CH(CH_3)_2)\text{-}CONH\text{-}CH(CH_3)COOCH_2Phe$

Le disulfure mixte pyridine / tBoc (S - benzyl) cystéinethiol (exemple 11, étape 7) (1 g) est condensé dans les conditions de l'exemple 1, étape 2, avec 1,07 g de N(mercapto méthyl-2 méthyl-4 oxo-1-pentyl)-alaninate de benzyle. On obtient 1,34 g (Rdt 65 %) du composé final protégé sur la fonction amine. Rf (cyclohexane/EtOAc/AcOH = 7/3/0,5) = 0,42. Le traitement par le TFA (exemple 1 étape 3) libère le composé final (0,99 g) (Rdt 86 %) Rf ($CH_2Cl_2$/MeOH = 8/2) = 0,35.

Exemple 18

Préparation du $H_2N-CH(CH_2S(O)CH_2Phe)CH_2S-S-CH_2CH-(CH_2CH(CH_3)_2)-CONHCH(CH_3)COOCH_2Phe$

Le composé final de l'exemple 17 protégé sur la fonction amine est oxydé dans les conditions décrites pour l'exemple 8. Après déprotection par le TFA (exemple 1, étape 3), on obtient le composé final attendu. Rdt global (73 %) Rf ($CH_2Cl_2$/MeOH = 7/3) = 0,52.

Exemple 19

Préparation du $H_2N-CH(CH_2StBu)CH_2S-S-CH_2CH-(CH_2CH(CH_3)_2)-CONH-CH(CH_3)COOCH_2Phe$

A partir de 0,93 g de t Boc-S-t.butyl cystéinethiol, on obtient dans les conditions de l'exemple 1, étape 1, 1,06 g (Rdt 80 %) de disulfure mixte pyridine, tBoc(StBu) cystéinethiol. Ce composé est ensuite condensé dans les conditions de l'exemple 1 étape 2 avec 0,86 g de N(mercapto-méthyl-2 méthyl-4 oxo-1 pentyl) alaninate de benzyle. On obtient 1,19 g du composé final protégé (Rdt 75 %). Rf (cyclohexane/EtOAc/AcOH = 7/3/0,5) = 0,31. Le traitement par le TFA libère le composé final (0,84 g) (Rdt 85 %) Rf ($CH_2Cl_2$/MeOH = 7/3) = 0,38.

Exemple 20

Préparation du $H_2N-CH(CH_2S(O)tBu)CH_2S-S-CH_2CH-(CH_2CH)CH_3)_2)-CONHCH(CH_3)COOCH_2Phe$

Le composé de l'exemple 19 protégé sur la fonction amine est oxydé dans les conditions décrites pour l'exemple 8. Après déprotection par le TFA (exemple 1 étape 3) on obtient le composé final attendu. (Rdt global 68 %) Rf ($CH_2Cl_2$/MeOH = 7/3) = 0,30.

Exemple 21

Préparation du $H_2NCH(CH_2CH_2SCH_3)CH_2-S-S-CH_2-CH-(CH_2CH_2SCH_3)CONHCH(CH_3)COOCH_2Phe$

1,71 g du disulfure mixte pyridine/tBoc méthioninethiol (exemple 5, étape 1) est condensé dans les conditions de l'exemple 1 étape 2 avec 1,70 g de N(mercapto-méthyl-2-méthylmercapto-4oxo-1 butyl) alaninate de benzyle. On obtient 2,1 g (72 %) du composé final protégé sur la fonction amine. Rf (cyclohexane/EtOAc/AcOH = 7/3/0,5) 0,58. Le traitement par le TFA (exemple 1, étape 3) libère le composé final (1,18 g) (Rdt 68 %) Rf ($CH_2Cl_2$/MeOH : 7/3) :0,42.

Exemple 22

Préparation du $H_2N-CH(CH_2CH_2S(O)CH_3)CH_2-S-S-CH_2CH(CH_2CH_2S(O)CH_3)CONHCH(CH_3)COOCH_2Phe$

Le composé final de l'exemple 21 protégé sur la fonction amine est oxydé dans les conditions décrites pour l'exemple 8. Après déprotection par le TFA (exemple 1, étape 3), on obtient le composé final attendu. Rdt global 56 %.

Exemple 23

Préparation du $H_2NCH(CH_2CH_2SCH_3)CH_2-S-S-CH_2-CH(CH_2Phe)CONH-CH(Phe)CH_2COOCH_2Phe$

1,19 g du disulfure mixte pyridine N-Boc méthioninethiol (exemple 5, étape 1) est condensé dans les conditions de l'exemple 1 étape 2 avec 1,44 g de N(mercaptométhyl-2 phényl-3 oxo-1 propyl)-3-phényl béta-alaninate de benzyle. On obtient 1,59 g (Rdt 70 %) du composé final protégé sur la fonction amine. Rf (cyclohexane/EtOAc/AcOH = 7/3/0,5) : 0,28. Le traitement par le TFA (exemple 1, étape 3) libère le composé final (1,15 g) (Rdt 85 %) Rf ($CH_2Cl_2$/MeOH : 7/3) = 0,60.

Exemple 24

Préparation du $H_2NCH(CH_2CH_2S(O)CH_3)CH_2$-S-S-$CH_2CH(CH_2Phe)CONH$ $CH(Phe)CH_2COOCH_2Phe$

Le composé final de l'exemple 23 protégé sur la fonction amine est oxydé dans les conditions décrites pour l'exemple 8. Après déprotection par le TFA (exemple 1, étape 3), on obtient le composé final attendu. (Rdt global 58 %). Rf ($CH_2Cl_2$/MeOH : 7/3) = 0,25.

Exemple 25

Préparation du $H_2NCH(CH_2CH_2SCH_3)CH_2$-S-S-$CH_2$-$CH(CH_2Phe)$ CONH-CH—CH— $COOCH_2Phe$

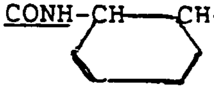

0,895 g du disulfure mixte pyridine N-Boc méthionine thiol (exemple 5 étape 1) est condensé dans les conditions de l'exemple 1 étape 2 avec 1,027 g de N(mercapto-méthyl-2-phényl-3-oxo-1-propyl)-amino-2-cyclohexyl-carbo-xylate de benzyle. On obtient 1,12 g (Rdt 68 %) du composé final protégé sur la fonction amine. Rf (cyclohexane/EtOAc/AcOH = 7/3/0,5) = 0,32. Le traitement par le TFA (exemple 1, étape 3) libère le composé final (0,78 g) (Rdt 82 %) Rf ($CH_2Cl_2$/MeOH : 7/3) : 0,63.

Exemple 26

Préparation du $H_2NCH(CH_2CH_2S(O)CH_3)CH_2$-S-S-$CH_2CH(CH_2Phe)$ CONH—CH—CH - $COOCH_2Phe$

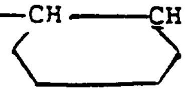

Le composé final de l'exemple 25 protégé sur la fonction amine est oxydé dans les conditions décrites dans l'exemple 8. Après déprotection par le TFA (exemple 1, étape 3), on obtient le composé attendu. Rdt global 62 %. Rf ($CH_2Cl_2$/MeOH : 8/2) : 0,28.

Exemple 27

Préparation du $H_2N$-$CH(CH_2CH_2SCH_3)$-$CH_2$-S-S-$CH_2$-$CH(CH_2CH_2SCH_3)$ CONH-CH(Phe)-$CH_2$-$COOCH_2Phe$

1,1 g du disulfure mixte pyridine N-Boc méthionine thiol (exemple 5, étape 1) sont condensés dans les conditions de l'exemple 1 étape 2 avec 1,39 g de N(mercaptométhyl-2-méthyl mercapto-4-oxo-1-butyl)-amino-3-phényl-3 propanoate de benzyle. On obtient 1,44 g (Rdt 65 %) du composé final protégé sur la fonction amine. Rf (cyclohexane/EtOAc/AcOH = 7/3/0,5) = 0,42. Le traitement par le TFA (exemple 1, étape 3) libère le composé final (1,01 g) (Rdt 85 %) Rf ($CH_2Cl_2$/MeOH : 7/3) : 0,72.

Exemple 28

Préparation du $H_2N$-$CH(CH_2CH_2S(O)CH_3)CH_2$-S-S-$CH_2$-$CH(CH_2CH_2S(O)CH_3)$CONH-CH(Phe)-$CH_2$-$COOCH_2Phe$

Le composé final de l'exemple 27 protégé sur la fonction amine est oxydé dans les conditions de l'exemple 4. Après déprotection par le TFA (exemple 1, étape 3), on obtient le composé attendu. Rdt global 53 %. Rf ($CH_2Cl_2$/MeOH : 8/2) : 0,35.

Exemple 29

Préparation de $H_2N-CH(CH_2CH_2SCH_3)CH_2-S-S-CH_2-CH(CH_2CH_2SCH_3)$ $CONH-CH(CH_2Phe)CH_2-COOCH_2Phe$

0,83 g du disulfure mixte pyridine N-Boc méthioninethiol (exemple 5 étape 1) est condensé dans les conditions de l'exemple 1 étape 2 avec 1,07 g de N(mercaptométhyl-2-méthyl mercapto-4-oxo-1-butyl)-amino-3-phényl-4 butanoate de benzyle. On obtient 1,22 g (72 %) du composé final protégé sur la fonction amine. Rf (cyclohexane/EtOAc/AcOH = 7/3/0,5) = 0,41. Le traitement par le TFA (exemple 1, étape 3) libère le composé final (0,79 g) (Rdt 72 %) Rf ($CH_2Cl_2$/MeOH : 7/3): 0,70.

Exemple 30

Préparation de $H_2N-CH(CH_2S(O)CH_3)-CH_2-S-S-CH_2-CH(CH_2CH_2S(O)CH_3)$ $CONH-CH(CH_2Phe)-CH_2-COOCH_2Phe$

Le composé final de l'exemple 29 protégé sur la fonction amine est oxydé dans les conditions de l'exemple 8. Après déprotection par le TFA (exemple 1 étape 3) on obtient le composé attendu. Rdt global 68 %. Rf ($CH_2Cl_2$/ MeOH : 8/2) : 0,33.

Exemple 31

Préparation de $H_2N-CH(CH_2CH_2SCH_3)CH_2-S-S-CH_2-CH(CH_2CH_2SCH_3)$
CONH-CH CH - $COOCH_2Phe$

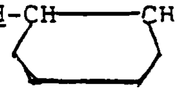

0,95 g du disulfure mixte pyridine N-Boc méthioninethiol (exemple 5 étape 1) est condensé dans les conditions de l'exemple 1 étape 2 avec 1,13 g de N(mercaptométhyl-2-méthyl mercapto-4-oxo-1-butyl)-amino-2-cyclohexyle carboxylate de benzyle. On obtient 1,43 g (78 %) du composé final protégé sur la fonction amine. Rf (cyclohexane/EtOAc/AcOH = 7/3/0,5) = 0,39. Le traitement par le TFA (exemple 1, étape 3) libère le composé final (0,88 g) (Rdt 75 %) Rf ($CH_2Cl_2$/MeOH : 7/3) : 0,65.

Exemple 32

Préparation du $H_2N-CH(CH_2CH_2S(O)CH_3)CH_2-S-S-CH_2-CH(CH_2CH_2-S(O)CH_3)$CONH-CH-CH-$COOCH_2Phe$

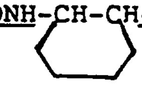

Le composé protégé sur la fonction amine de l'exemple 31 est oxydé dans les conditions de l'exemple 8. Après déprotection par le TFA (exemple 1 étape 3) on obtient le composé attendu. Rdt global 58 %. Rf ($CH_2Cl_2$/ MeOH : 8/2) : 0,30.

Exemple 33

Préparation de $H_2N-CH(CH_2CH_2S(O)CH_3)CH_2-S-S-CH_2-CH(CH_2CH(CH_3)_2)$
CONH-

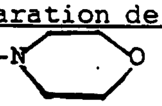

Le composé protégé sur la fonction amine de l'exemple 5, étape 1, est condensé sur le N-morpholinyl (isopropyl-méthyl)-3-mercapto-propanamide dans les conditions de l'exemple 5, étape 2. Le composé

obtenu est alors oxydé par le périodate de sodium puis est déprotégé par l'acide trifluoro-acétique dans les conditions de l'exemple 8 pour conduire au composé final. Rendement global: 62 %. Rf ($CH_2Cl_2$/MeOH = 8/2) = 0,12.

Exemple 34 Activité biologique des composés selon l'invention

L'activité biologique concerne l'inhibition de l'aminopeptidase M et de l'endopeptidase neutre purifiées.

1°) Inhibition in vitro des activités enkephalinasiques.

a) pouvoir inhibiteur sur l'endopeptidase neutre

Le pouvoir inhibiteur est déterminé sur de l'endopeptidase neutre purifiée du rein de lapin en suivant le protocole décrit dans la littérature (Blumberg et al. Life Sci. (1981) 28, 301). Après une préincubation de 15 minutes à 25°C, un aliquot de protéines est incubé pendant 20 minutes à 25°C en présence de 20 nmoles de ($^3$H)D-Ala$^2$-Leu$^5$-enkephaline et du composé à tester en solution dans le tampon tris HCl (pH 7,4) en présence de dithiothreitol DTT (100 équivalents par rapport à l'inhibiteur). On arrête la réaction par addition d'HCl 0,2 N. Le métabolite tritié ($^3$H)-Tyr-D-Ala-Gly est séparé de la D-Ala$^2$-Leu$^5$-enkephaline par chromatographie sur colonne de Porapak et la quantité formée mesurée à l'aide d'un compteur à scintillation liquide.

b) Pouvoir inhibiteur sur l'aminopeptidase N

Le pouvoir inhibiteur est mesuré sur l'aminopeptidase purifié du rein de porc (Boehringer, France). Le dosage est conduit de manière identique au précédent en utilisant la ($^3$H)-Leu$^5$-enképhaline comme substrat. Le métabolite formé ($^3$H) Tyr est séparé par chromatographie sur colonne de Porapak et la quantité formée mesurée à l'aide d'un compteur à scintillation liquide.

L'activité des différents composés est exprimée en concentrations inhibitrices à 50 % ($IC_{50}$) et les résultats sont résumés dans le Tableau II.

TABLEAU II

| | $IC_{50}$ Endopeptidase neutre | $IC_{50}$ Aminopeptidase N |
|---|---|---|
| Composé final de l'exemple 1 | $2.10^{-9}$ M | $5.10^{-9}$ M |
| Composé final de l'exemple 2 | $2,5.10^{-9}$ M | $5.10^{-9}$ M |
| Composé final de l'exemple 3 | $1,2.10^{-9}$ M | $5.10^{-9}$ M |
| Composé final de l'exemple 4 | $2.10^{-9}$ M | $8.10^{-9}$ M |
| Composé final de l'exemple 5 | $2.10^{-9}$ M | $2.10^{-9}$ M |
| Composé final de l'exemple 6 | $1,2.10^{-9}$ M | $2.10^{-9}$ M |
| Composé final de l'exemple 7 | $2,5.10^{-9}$ M | $2.10^{-9}$ M |
| Composé final de l'exemple 8 | $2.10^{-9}$ M | $2.10^{-9}$ M |
| Composé final de l'exemple 22 | $7.10^{-9}$ M | $2.10^{-9}$ M |
| Composé final de l'exemple 26 | $5.10^{-9}$ M | $2.10^{-9}$ M |
| Composé final de l'exemple 28 | $3.10^{-9}$ M | $2.10^{-9}$ M |
| Composé final de l'exemple 30 | $3.10^{-9}$ M | $2.10^{-9}$ M |
| Composé final de l'exemple 32 | $4.10^{-9}$ M | $2.10^{-9}$ M |
| Composé final de l'exemple 33 | $5.10^{-8}$ M | $2.10^{-9}$ M |

Des pouvoirs inhibiteurs identiques sont obtenus sur ces deux enzymes après incubation des composés avec des fractions membranaires de cerveau de rat, à la place du Dithiothreitol.

2°) Inhibition "in vivo" de la NEP cérébrale

On compare l'activité enzymatique de la NEP cérébrale chez la souris traitée ou non traitée par une dose donnée de composé (environ 2,5 mg/kg injecté par voir iv). Les animaux sont sacrifiés à des temps variables après l'injection de l'inhibiteur et l'activité NEP est déterminée dans le cerveau par mesure de la dégradation de la ($^3$H) DAla$^2$-Leu$^5$ enképhaline.

Pour le composé final de l'exemple 1, injecté à la dose de 2,7 µmole/kg (47,6 µg dans 100 µl), on obtient les résultats suivants :

| temps | % inhibition NEP cérébrale |
| --- | --- |
| 15' (n = 4) | 96 ± 9 |
| 60' (n = 4) | 68 ± 4 |
| 120' (n = 4) | 63 ± 2 |
| 180' (n = 4) | 42 ± 16 |

Pour le composé final de l'exemple 7, on obtient dans les mêmes conditions :

| temps | % inhibition NEP cérébrale |
| --- | --- |
| 60' (n = 4) | 96 ± 5 |
| 120' (n = 5) | 96 ± 4 |
| 180' (n = 5) | 92 ± 4 |

Exemple 35 Etude pharmacologique des composés

a) Toxicité aiquë

La détermination de la mortalité chez la souris est observée à la suite de l'administration unique par voie intraveineuse de doses croissantes des composés à tester. La DL50 pour tous les composés étudiés est supérieure à 100 mg/kg i.v.

b) Activité antalgique

1°) Test de la plaque chaude : Il concerne le réflexe de léchement et de saut de la souris sur une plaque chauffée à 55°C selon la méthode de Jacob et al. (Arch. Int. Pharmacodyn. 122 287. 1959 ; 1961).

1.1. Administrée par voie i.v. (100 µl dans la veine de la queue) chez la souris, les composés testés présentent un effet propre à la fois sur le temps de latence du saut et sur celui des léchements. Ces effets sont antagonisés par la naloxone (0,5 mg/kg s.c.).

Les résultats sont exprimés dans le Tableau III en % d'analgésie. Le pourcentage d'analgésie s'exprime pour chaque dose de composé testé par la relation :

$$\% \text{ analgésie} = \frac{(\text{temps de latence mesuré} - \text{temps de latence témoin})}{(\text{temps de latence maximum} - \text{temps de latence témoin})}$$

TABLEAU III

| Composés | 50 % analgésie | 50 % analgésie |
| --- | --- | --- |
| Composé final de l'exemple 1 | 4 mg/kg | 20 mg/kg |
| Composé final de l'exemple 5 | 2,5 mg/kg | 10 mg/kg |
| Composé final de l'exemple 7 | 5 mg/kg | 15 mg/kg |
| Composé final de l'exemple 8 | 2,5 mg/kg | 10 mg/kg |
| Composé final de l'exemple 22 | 4,5 mg/kg | 25 mg/kg |
| Composé final de l'exemple 26 | 8 mg/kg | 30 mg/kg |
| Composé final de l'exemple 28 | 4 mg/kg | 15 mg/kg |
| Composé final de l'exemple 30 | 3 mg/kg | 15 mg/kg |
| Composé final de l'exemple 32 | 2 mg/kg | 10 mg/kg |

1.2 Administrés par voie i.p. chez la souris, les composés testés présentent un effet propre sur les temps de retenue du saut et des léchements. Ces effets sont antagonisés par la naloxone (0,5 mg/kg).

Les résultats sont exprimés en ED 50

| | |
|---|---|
| Composé de l'exemple 1 | 10 mg/kg |
| Composé de l'exemple 5 | 7 mg/kg |
| Composé de l'exemple 7 | 8 mg/kg |
| Composé de l'exemple 22 | 6 mg/kg |
| Composé de l'exemple 32 | 5,5 mg/kg |

2°) Essai sur les crampes abdominales

(Hendershot et Forsaith. J. Pharmacol. (1975), 125, 237) : Ce test mesure le nombre de crampes induites par injection de 100 µl d'une solution à 0,02 % de phénylbenzoquinone par voie i.p. Le nombre de crampes est compté pendant 10 minutes, 10 minutes après l'injection. Administrés par voie i.v. les composés diminuent le nombre de crampes.

| | dose | % analgésie |
|---|---|---|
| Composé final de l'exemple 1 | 1 mg/kg | 24,6 % ± 8 |
| Composé final de l'exemple 1 | 3 mg/kg | 46,9 % ± 7,9 |
| Composé final de l'exemple 1 | 9 mg/kg | 73,1 % ± 8,2 |
| Composé final de l'exemple 7 | 2,5 mg/kg | 32 % ± 4 |
| Composé final de l'exemple 7 | 5 mg/kg | 68 % ± 9 |
| Composé final de l'exemple 7 | 10 mg/kg | 95 % ± 4 |

3°) Essai sur le retrait de la queue chez le rat : (tail flick test) : Administrés par voie i.v. chez le rat, les composés selon l'invention produisent un allongement significatif du temps de retrait de la queue. Ces effets sont antagonisés par la naloxone (0,5 mg/kg s.c.).

Les résultats sont exprimés en % d'analgésie pour une même dose (10 mg/kg) de tous les composés testés.

| | % d'analgésie |
|---|---|
| Composé de l'exemple 1 | 20 % |
| Composé de l'exemple 5 | 25 % |
| Composé de l'exemple 7 | 42 % |
| Composé de l'exemple 32 | 36 % |

c) Potentialisation des effets comportementaux (hyperactivité locomotrice) induits par les composés selon l'invention après traitement chronique aux antidépresseurs :

Les composés selon l'invention induisent, 1 h après administration i.v. ou p.o. chez le rat, une augmentation de l'activité locomotrice. Cet effet est considérablement augmenté après traitement chronique par des neuroleptiques tel le sulpiride (100 mg/kg) i.p. chaque jour). Cette potentialisation n'apparaît que trois semaines après le début du traitement par les neuroleptiques. Les résultats montrent une augmentation de la concentration en enképhalines endogènes, en accord avec les résultats obtenus avec la morphine par Stinus et Coll. (psychoparmacology, 85, 323, (1985).

| Résultats* | Activité locomotrice** |
|---|---|
| rat témoin*** | 100 % |
| rat traité par le composé de l'exemple 5 i.v. (5 mg/kg) | 400 % |
| rat traité par le composé de l'exemple 7 i.v. (3 mg/kg) | 400 % |
| rat traité par le composé de l'exemple 30 i.v. (2 mg/kg) | 350 % |
| rat traité par le composé de l'exemple 30 i.v. (20 mg/kg) | 370 % |

* après trois semaines de traitement chronique au sulpiride
** mesurée par le nombre de redressements
*** correspond aux animaux traités chroniquement par le sulpiride, mais n'ayant pas reçu d'inhibiteur.

d) Test du "désespoir" chez la souris

Ce test décrit par Porsolt et call. (Arch. Int. Pharmacodyn. Ther, 229, 327, (1977), permet d'évaluer les propriétés antidépressives des composés revendiqués.

Les souris sont traitées par des doses croissantes de composés trente minutes avant le test et l'immobilité des animaux est mesurée par rapport à des témoins qui perçoivent seulement le véhicule. Le temps d'immobilité est diminué par les composés selon l'invention et cet effet est antagonisé par la naxolone (0,5 mg/kg s.c.).

| Résultats | Temps d'immobilité (s) |
|---|---|
| témoins | 165 ± 15 |
| souris traitée par composé ex. 5 | |
| ( 5 mg/kg i.p.) | 108 ± 12* |
| souris traitée par composé ex. 30 | |
| - ( 2 mg/kg i.p.)<br>- (20 mg/kg p.o.) | 96 ± 8*<br>101 ± 10* |

* $p < 0,01$.

e) Etude des effets de tolérance et de dépendance induits bar administration chronique du composé de l'exemple 8 :

1) Le composé 8 est administré quatre fois par 24 heures à la dose de 5 mg/kg par voie s.c. durant 6 jours à des souris et à 10 mg/kg à des rats.

La morphine (chlorhydrate) est administrée dans les mêmes conditions (0,5 mg/kg chez la souris, 2 mg/kg chez le rat). Les animaux témoins reçoivent uniquement le véhicule.

| Résultats en % d'analgésie par rapport aux témoins | | | |
|---|---|---|---|
| jours | 0 | 1 | 6 |
| souris/morphine | 100 ± 10 | 60 ± 10 | 0 |
| rat/morphine | 70 ± 12 | 20 ± 3 | 0 |
| souris/composé 8 | 100 ± 10 | 100 ± 8 | 70 ± 15 |
| rat/composé 8 | 40 ± 5 | 40 ± 5 | 30 ± 7 |
| rat/composé 7 | 100 ± 10 | 100 ± 8 | 80 ± 10 |

2) Après traitement chronique durant 6 jours, dans les conditions décrites précédemment, les rats subissent une injection de 2 mg/kg de naxolone par voie s.c. Les signes du syndrome d'abstinence sont mesurés selon Cowan et al. (J. Pharmacol. Exp. Ther. (1988) 246, 950).

Par rapport à la morphine, le traitement chronique avec le composé 8 engendre des effets de dépendance très faibles illustrés par l'absence de sauts, l'absence de baisse de température, l'absence de perte de poils qui sont les signes les plus sévères de la crise d'assuétude.

3) L'injection i.v. du composé 7 à un animal (souris ou rat) tolérant à la morphine conduit à une réponse analgésique identique à celle obtenue chez un animal témoin. Ceci démontre l'absence de tolérance croisée entre la morphine et le composé 7.

L'invention a d'autre part pour objet des procédés de traitement de la dépression et de la douleur dans lesquels on administre des quantités efficaces d'au moins un des composés précédemment décrits au sujet à traiter. Les voies d'administration convenant auxdits procédés sont celles usuelles pour ces types de traitement, en particulier intraveineuse, per os, sous-cutanée ou intrapéritonéale.

**Revendications**

**Revendications pour les Etats contractants suivants : AT, BE, CH, DE, DK, FR, GB, IT, LI, LU, NL, SE**

**1.** Composés chimiques répondant à la formule :

$H_2N\text{-}CH(R_1)\text{-}CH(R_2)\text{-}S\text{-}S\text{-}CH(R_3)\text{-}CH(R_4)\text{-}A\text{-}B\text{-}Z$ (I)

dans laquelle

$R_1$ représente :

- une chaîne alkylée hydrocarbonée saturée ou insaturée linéaire ou ramifiée comportant de 1 à 6 atomes de carbone, substituée ou non par un groupe OR dans laquelle R est un hydrogène, une chaîne hydrocarbonée linéaire ou ramifiée de C1 à C4, ou un reste phényle ou benzyle ; un groupe thioéther SR, R ayant la même définition que ci-dessus ou un groupe thioéther oxydé S-(O)R, R ayant la même définition que ci-dessus,
- un groupe méthyl cycloalkyle à 5 à 6 atomes de carbone, un groupe benzyl ou phényl, éventuellement substitué :
  . par 1 à 5 atomes d'halogène notamment de fluor,
  . ou par un groupe OR' ou SR', R' étant un groupement alkylé de 1 à 4 atomes de carbone, le thioéther SR' étant oxydé ou non sur l'atome de soufre,
  . ou par un groupe amino éventuellement mono ou disubstitué par un groupe alkyle aliphatique de 1 à 6 atomes de carbone, oxydé ou non sur la fonction amine,
- un groupe méthylène substitué par un hétérocycle à 5 ou 6 atomes de carbone, aromatique ou saturé, possédant comme hétéroatome, un atome d'azote, un atome d'oxygène ou un atome de soufre, les atomes d'azote et de soufre étant éventuellement oxydés sous forme de N-oxyde ou de S-oxyde.

$R_2$ représente indépendamment, un atome d'hydrogène ou un groupement méthyle.

$R_3$ représente un atome d'hydrogène ou une chaîne alkyle linéaire ou ramifiée.

$R_4$ représente :

- une chaîne hydrocarbonée saturée ou insaturée, linéaire ou ramifiée comportant de 1 à 6 atomes de carbone, substitué ou non par un groupe hydroxy, éther OR dans lequel le groupe R a la même signification que précédemment, un groupe thiol SH, thioether SR (R ayant la même signification que précédemment) ou un groupe thioether oxydé en sulfoxyde,
- ou un groupe méthylcycloalkyle de 5 à 6 atomes de carbone, un groupe benzyl ou phényl, éventuellement substi-tué par 1 à 5 atomes d'halogène notamment de fluor, par un hydroxyle ou un thiol, un éther ou un thioether OR' ou SR', R' ayant de 1 à 4 atomes de carbone, le thioether étant oxydé ou non sur l'atome de soufre, par un groupe amino éventuellement mono ou disubstitué par un groupe alkyle aliphatique de 1 à 6 atomes de carbone, oxydé ou non sur la fonction amine,
- ou un groupe méthylène substitué par un hétérocyle à 5 ou 6 atomes de carbone, aromatique ou saturé, possédant comme hétéroatome, un atome d'azote, un atome d'oxygène ou un atome de soufre, les atomes d'azote et de soufre étant éventuellement oxydés sous forme de N-oxyde ou de S-oxyde.

Z peut représenter:

* un groupement $-CH(R_5)-[CH(R_6)]_n-COO(R_7)$

dans lequel

$R_5$ et $R_6$ peuvent représenter indépendamment l'un de l'autre: . un hydrogène,

. ou un groupement alkyle linéaire ou ramifié de 1 à 6 atomes de carbone, substitué ou non par des groupements hydroxy ou éther OR, thiol SH ou thioether, SR (R ayant la définition précisée précédemment),

. ou un groupement phényl (Phe) ou benzyl substitué ou non par 1 à 5 atomes d'halogène ; un groupement OR' ou SR', R' ayant la définition précisé ci-dessus.

$R_5$ et $R_6$ peuvent également représenter chacun des chaînes hydrocarbonées saturées ou insaturées contenant de 1 à 6 atomes de carbone dans lesquels 1 à 2 atomes de carbone peuvent être substitués par un atome d'oxygène, de soufre ou d'azote pouvant conduire à la formation de un ou plusieurs cycles d'hydrocarbones saturés ou aromatiques de 5 à 6 maillons ou d'hétérocycles saturés ou aromatiques de 5 à 6 maillons.

n peut être égal à 0 ou à 1

$R_7$ représente :

. un hydrogène

. ou un groupement alkyle linéaire ou ramifié de 1 à 6 atomes de carbone saturé ou insaturé

. ou un groupement benzyle.

* un groupement morpholinyle $-N-C_4H_8O$

* un groupement piperidinyle $-N\ C_5H_{10}$, éventuellement substitué par un reste -OH, un reste $-CO_2H$, un reste $COOR^1$, dans lequel $R^1$ représente une chaîne alkyle linéaire ou ramifiée contenant 1 à 6 atomes de carbone, un reste phényle ou un reste benzyle.

* un groupement pyralozinyle-$N-C_4H_8-NH$, un groupement pyralozinyle substitué $-NC_4H_8-N-R^2$ dans lequel $R^2$ représente une chaîne alkylée contenant de 1 à 6 atomes de carbone, un reste benzyle ou un reste phényle.

A-B représente un groupement amide CONH ou rétro amide NHCO lesdits composés se présentant sous formes de mélanges racémiques, sous formes d'énantiomères, de diastéréoisomère ou de stéréoisomères ainsi que leurs mélanges et leurs sels pharmaceutiquement acceptables.

**2.** Composés selon la revendication 1, caractérisés en ce que Z représente un groupement

$-CH(R_5)-[CH(R_6)]_n\ COO(R_7)$,

$R_2$ et $R_3$ représentent tous deux des atomes d'hy-drogène, AB représente une liaison amide CONH, $R_1$ représente un groupement $CH_2CH_2\ S(O)CH_3$ et $R_7$ représente un groupement $CH_2Phe$.

**3.** Composés selon la revendication 2, caractérisés en ce que $R_4$ représente $-CH_2-CH_2S(O)CH_3$.

**4.** Composé selon la revendication 2, répondant à la formule :

$H_2N-CH(CH_2CH_2S(O)CH_3)-CH_2S-S-CH_2-CH(CH_2Phe)CONHCH_2COOCH_2Phe$

**5.** Composé selon la revendication 2, répondant à la formule :

$H_2NCH(CH_2CH_2S(O)CH_3)CH_2-S-S-CH_2-CH(CH_2Phe)CONH-$ 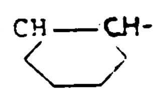

$COOCH_2Phe$

**6.** Composé selon la revendication 3, répondant à la formule :

$H_2N-CH(CH_2CH_2S(O)CH_3)CH_2-S-S-CH_2CH(CH_2CH_2S(O)CH_3)CONHCH(CH_3)$
$COOCH_2Phe$

**7.** Composé selon la revendication 3, répondant à la formule :

$H_2N-CH(CH_2CH_2S(O)CH_3)-CH_2-S-S-CH_2-CH(CH_2CH_2S(O)CH_3)CONH-$
$CH(Phe)-CH_2-COOCH_2Phe$

**8.** Composé selon la revendication 3, répondant à la formule :

$H_2N-CH(CH_2CH_2S(O)CH_3)-CH_2-S-S-CH_2-CH(CH_2CH_2S(O)CH_3)CONH-$
$CH(CH_2Phe)-CH_2-COOCH_2Phe$

**9.** Composé selon la revendication 3, répondant à la formule :

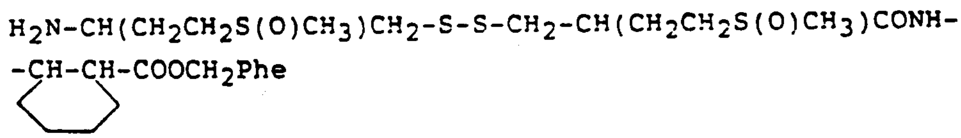

**10.** Composé selon la revendication 1, répondant à la formule :

$H_2NCH(CH_2CH_2SCH_3)CH_2S\text{-}S\text{-}CH_2CH(CH_2CH(CH_3)_2)CONHCH(CH_3)$
$COOCH_2Phe$

**11.** Composé selon la revendication 1, répondant à la formule :

$H_2NCH(CH_2CH_2SCH_3)CH_2\text{-}S\text{-}S\text{-}CH_2\text{-}CH\ (CH_2CH_2SCH_3)CONHCH(CH_3)$
$COOCH_2Phe$

**12.** Composé selon la revendication 1, répondant à la formule :

$H_2N\text{-}CH(CH_2CH(CH_3)_2)\text{-}CH_2\text{-}S\text{-}S\text{-}CH_2\text{-}CH(CH_2Phe)CONH\text{-}CH_2COOCH_2Phe$

**13.** Composé selon la revendication 1, répondant à la formule :

$H_2N\text{-}CH(CH_2CH(CH_3)_2)\text{-}CH_2\text{-}S\text{-}S\text{-}CH_2\text{-}CH(CH_2Phe)CONH\text{-}CH(CH_3)COOCH_2Phe$.

**14.** Composé selon la revendication 1, répondant à la formule :

$H_2N\text{-}CH(CH_2CH(CH_3)_2)\text{-}CH_2\text{-}S\text{-}S\text{-}CH_2\text{-}CH(CH_2Phe)CONH\text{-}CH(CH_2Phe)\text{-}$
$COOCH_2Phe$.

**15.** Composé selon la revendication 1, répondant à la formule :

$H_2N\text{-}CH(CH_2CH_2SCH_3)\text{-}CH_2\text{-}S\text{-}S\text{-}CH_2\text{-}CH\ (CH_2Phe)CONH\text{-}CH_2\text{-}COOCH_2Phe$.

**16.** Composé selon la revendication 1, répondant à la formule :

$H_2N\text{-}CH(CH_2CH_2SCH_3)\text{-}CH_2\text{-}S\text{-}S\text{-}CH_2\text{-}CH(CH_2Phe)CONH\text{-}CH(CH_3)\text{-}COOCH_2Phe$.

**17.** Composé selon la revendication 1, répondant à la formule :

$H_2N\text{-}CH(CH_2CH_2SCH_3)CH_2\text{-}S\text{-}S\text{-}CH_2\text{-}CH\ (CH_2Phe)CONH\text{-}CH(CH_2Phe)\text{-}$
$COOCH_2Phe$.

**18.** Composé selon la revendication 1, répondant à la formule :

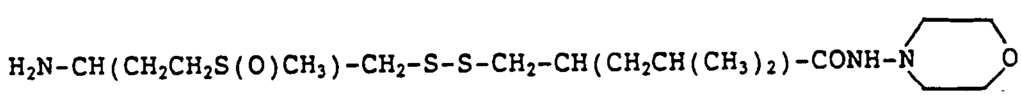

**19.** Composé selon la revendication 1, répondant à la formule :

$H_2N\text{-}CH(CH_2CH_2S(O)CH_3)\text{-}CH_2\text{-}S\text{-}S\text{-}CH_2\text{-}CH(CH_2Phe)\text{-}CONH\text{-}CH(CH_2$
$Phe)COOCH_2Phe$.

**20.** Procédé de préparation des composés de formule I dans laquelle Z représente un groupement

$-CH(R_5)-[CH(R_6)]_n-COO(R_7)$

caractérisé en ce que des composés de formule II :

$R_8NH-CH(R_1)-CH(R_2)SH$

dans laquelle $R_8$ représente un groupement protecteur de la fonction amine sont condensés sur la dithiodipyridine (III) pour conduire aux composés de formule générale (IV) :

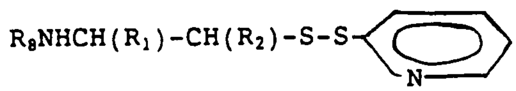

- puis le composé (IV) est condensé sur les composés de formule (V) :

  $HS-CH(R_3)-CH(R_4)-A-B-CH(R_5)-[(CH(R_6)]_n-COO(R_7)$

  pour conduire aux composés de formule (VI) :

  $R_8NH-CH(R_1)-CH(R_2)-S-S-CH(R_3)-CH(R_4)-A-B-CH(R_5)-[(CH(R_6)]_n-COO(R_7)$

- puis la fonction amine du composé (VI) est déprotégée pour conduire au composé de formule (I).

**21.** Procédé suivant la revendication 20, caractérisé en ce que, si $R_2$ représ ente un atome d'hydrogène, le produit de formule II est obtenu à partir d'un bêta-amino alcool de formule :

$H_2N-CH(R_1)-CH_2OH$ (VII)

- par protection de la fonction amine à l'aide d'un groupe $R_8$ résultant en la formation du produit de formule :

  $R_8NH-CH(R_1) - CH_2OH$ (VIII)

- puis par activation de la fonction alcool, notamment sous la forme de tosylate, résultant en la formation d'un produit de formule :

  $R_8HN-CH(R_1)\ CH_2OT_S$ (IX)

  dans laquelle $T_S$ représente un groupement tosylate,
- puis par transformation du groupement tosylate en groupement acétylthio résultant en la formation du produit de formule :

  $R_8NH - CH\ (R_1)\ CH_2-S-COCH_3$ (X)

- puis par hydrolyse du produit (X) résultant en la formation du composé de formule (II).

**22.** Procédé selon la revendication 20, caractérisé en ce que si $R_2$ représente un atome d'hydrogène, le produit de formule (X) est obtenu à partir du produit de formule (VIII), en présence de triphényl phosphine et d'un azodicarboxylate de dialkyle.

**23.** Procédé selon la revendication 20, caractérisé en ce que si $R_2$ représente un groupe $-CH_3$, et si $R_1$ et $R_4$ ont la même signification, le composé de formule (II) est obtenu à partir d'un composé de formule :

$CH_3-CO-SCH(CH_3)CHR_1-CO_2H$ (XI)

- par transformation dudit composé en un produit de formule :

$CH_3$-CO-SCH($CH_3$)$CHR_1$-$CON_3$ (XII)

puis en un produit de formule :

$CH_3$-CO-SCH($CH_3$)$CHR_1$-$NHR_8$ (XIII)

- puis par hydrolyse acide du groupement acétylthio.

**24.** Procédé selon la revendication 20, caractérisé en ce que si $R_3$ représente un atome d'hydrogène ou un groupe $CH_3$, et AB un groupement amide CONH, le composé de formule (V) est obtenu à partir d'un composé de formule :

$CH_3$-CO-SCH($R_3$)CH($R_4$)-$CO_2$H (XIV)

- par hydrolyse alcaline dudit composé résultant en la formation du composé de formule :

(S CH($R_3$)CH($R_4$)-$CO_2$H$)_2$ (XV)

- puis condensation avec un amino-ester de formule :

$H_2$N-CH($R_5$)-[CH($R_6$)$]_n$-CO-$OR_7$ (XVI)

résultant en la formation d'un composé de formule :

(S $CHR_3$CH($R_4$)CONH CH($R_5$)[CH($R_6$)$]_4$-$COOR_7$$)_2$ (XVII)

- puis traitement en milieu réducteur.

**25.** Procédé selon la revendication 20, caractérisé en ce que le composé de base de formule XI du procédé selon la revendication 23 peut être obtenu à partir d'un composé de formule :

$(Phe)_3$P-CH($R_1$)-$COOCH_3$ (XVIII)

- par condensation avec de l'acétaldéhyde résultant en la formation du composé de formule :

$CH_3$CH = C($R_1$)-$COOCH_3$ (XIX)

- puis par hydrolyse alcaline résultant en la formation du composé de formule :

$CH_3$CH = C($R_1$)-$CO_2$H (XX)

- puis par addition d'acide thioacétique.

**26.** Procédé selon la revendication 20, caractérisé en ce que si $R_3$ représente un atome d'hydrogène ou un groupe -$CH_3$ et si $R_1$ et $R_4$ ont la même signification, et si AB représente un groupe rétro-amide NHCO, le composé de formule (V) est préparé respectivement à partir des composés (X) et (XIII) répondant à la formule générale :

$CH_3$CO S CH($R_2$)CH($R_1$)$NHR_8$ (XXI)

- par hydrolyse alcaline résultant en la formation du composé de formule :

[SCH($R_2$)CH($R_1$)$NHR_8$$]_2$ (XXII)

- puis par déprotection de la fonction amine conduisant au composé de formule :

$[SCH(R_2)CH(R_1)NH_2]_2$ (XXIII)

- puis par condensation avec des monoacides esters maloniques ou succiniques de formule :

$CO_2H\text{-}CH(R_5)\text{-}[CH(R_6)]_n\text{-}COOR_7$ (XXIV)

résultant en la formation d'un composé de formule :

$[SCH(R_2)CHR_1\text{-}NHCO\text{-}CHR_5\text{-}(CHR_6)_n\text{-}COOR_7]_2$

- puis par traitement en milieu réducteur.

**27.** Procédé de préparation des composés de formule I dans laquelle le groupement Z représente :
* un groupement morpholinyle $-N\text{-}C_4H_8O$
* un groupement piperidinyle $-N\ C_5H_{10}$, éventuellement substitué par un reste -OH, un reste $-CO_2H$, un reste $COOR^1$, dans lequel $R^1$ représente une chaîne alkyle linéaire ou ramifiée contenant 1 à 6 atomes de carbone, un reste phényle ou un reste benzyle
* un groupement pyralozinyle-$N\text{-}C_4H_8\text{-}N\text{-}R^2$ dans lequel $R^2$ représente une chaîne alkylée contenant de 1 à 6 atomes de carbone, un reste benzyle ou un reste phényle,

caractérisé en ce que des composés de formule :

$(SCH(R_3)CH(R_4)\text{-}CO_2H)_2$ (XV)

sont condensés avec une hydrazine de formule

$H_2N\text{-}Z$ (XXV)

résultant en la formation d'un composé de formule :

$(SCH(R_3)CH(R_4)\text{-}CONH\text{-}Z)_2$ (XXVI)

qui est condensé sur un composé de formule :

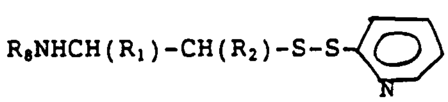 (IV)

pour conduire au composé de formule (I).

**28.** Composition pharmaceutique, caractérisée en ce qu'elle contient au moins un composé selon l'une des revendications 1 à 19 ou obtenu par un procédé selon l'une quelconque des revendications 2O à 27, en association avec un ou plusieurs diluants ou adjuvants compatibles ou pharmaceutiquement acceptables.

**29.** Composition pharmaceutique selon la revendication 28, caractérisée en ce qu'elle est destinée au traitement de la dépression et de la douleur.

**30.** Composition selon l'une des revendications 28 et 29 destinée à l'administration intraveineuse, per os, sous-cutanée ou intrapéritonéale.

**31.** Utilisation des composés selon l'une des revendications 1 à 19 ou obtenus par un procédé selon l'une des revendications 2O à 27 pour la fabrication d'un médicament pour le traitement de la dépression.

**32.** Utilisation des composés selon l'une des revendications 1 à 19 ou obtenus par un procédé selon l'une des revendications 2O à 27 pour la fabrication d'un médicament pour le traitement de la douleur.

**Revendications pour l'Etat contractant suivant : ES**

**1.** Procédé de préparation des composés de formule I :

$H_2N-CH(R_1)-CH(R_2)-S-S-CH(R_3)-CH(R_4)-A-B-Z$ (I)

dans laquelle :

$R_1$ représente :

- une chaîne alkylée hydrocarbonée saturée ou insaturée linéaire ou ramifiée comportant de 1 à 6 atomes de carbone, substituée ou non par un groupe OR dans laquelle R est un hydrogène, une chaîne hydrocarbonée linéaire ou ramifiée de $C_1$ à $C_4$, ou un reste phényle ou benzyle; un groupe thioéther SR, R ayant la même définition que ci-dessus ou un groupe thioéther oxydé S-(O)R, R ayant la même définition que ci-dessus,
- un groupe méthyl cycloalkyle à 5 à 6 atomes de carbone, un groupe benzyl ou phényl, éventuellement substitué:
  . par 1 à 5 atomes d'halogène notamment de fluor,
  . ou par un groupe OR' ou SR', R' étant un groupement alkylé de 1 à 4 atomes de carbone, le thioéther SR' étant oxydé ou non sur l'atome de soufre,
  . ou par un groupe amino éventuellement mono ou disubstitué par un groupe alkyle aliphatique de 1 à 6 atomes de carbone, oxydé ou non sur la fonction amine,
- un groupe méthylène substitué par un hétérocycle à 5 ou 6 atomes de carbone, aromatique ou saturé, possédant comme hétéroatome, un atome d'azote, un atome d'oxygène ou un atome de soufre, les atomes d'azote et de soufre étant éventuellement oxydés sous forme de N-oxyde ou de S-oxyde.

$R_2$ représente indépendamment, un atome d'hydrogène ou un groupement méthyle

$R_3$ représente un atome d'hydrogène ou une chaîne alkyle linéaire ou ramifiée

$R_4$ représente :

- une chaîne hydrocarbonée saturée ou insaturée, linéaire ou ramifiée comportant de 1 à 6 atomes de carbone, substitué ou non par un groupe hydroxy, éther OR dans lequel le groupe R a la même signification que précédemment, un groupe thiol SH, thioéther SR ( R ayant la même signification que précédemment) ou un groupe thioéther oxydé en sulfoxyde,
- ou un groupe méthylcycloalkyle de 5 à 6 atomes de carbone, un groupe benzyl ou phényl, éventuellement substitué par 1 à 5 atomes d'halogène notamment de fluor, par un hydroxyle ou un thiol, un éther ou un thioéther OR'ou SR', R' ayant de 1 à 4 atomes de carbone, le thioéther étant oxydé ou non sur l'atome de soufre, par un groupe amino éventuellement mono ou disubstitué par un groupe alkyle aliphatique de 1 à 6 atomes de carbone, oxydé ou non sur la fonction amine,
- ou un groupe méthylène substitué par un hétérocycle à 5 ou 6 atomes de carbone, aromatique ou sature, possédant comme hétéroatome, un atome d'azote, un atome d'oxygène ou un atome de soufre, les atomes d'azote et de soufre étant éventuellement oxydés sous forme de N-oxyde ou de S-oxyde

  Z représente un groupement

  $-CH(R_5)-[CH(R_6)]_n-COO(R_7)$

  dans lequel:

  $R_5$ et $R_6$ peuvent représenter indépendamment l'un de l'autre:
  . un hydrogène,
  . ou un groupement alkyle linéaire ou ramifié de 1 à 6 atomes de carbone, substitué ou non par des groupements hydroxy ou éther OR, thiol SH ou thioéther, SR ( R ayant la définition précisée précédemment),
  . ou un groupement phényl (Phe) ou benzyl substitué ou non par 1 à 5 atomes d'halogène; un groupement OR' ou SR', R' ayant la définition précisée ci-dessus.

  $R_5$ et $R_6$ peuvent également représenter chacun des chaînes hydrocarbonées saturées ou insaturées contenant de 1 à 6 atomes de carbone dans lesquels 1 à 2 atomes de carbone peuvent être substitués par un atome d'oxygène, de soufre ou d'azote pouvant conduire à la formation de un ou plusieurs cycles d'hydrocarbones saturés ou aromatiques de 5 à 6 maillons ou d'hétérocycles saturés ou aromatiques de 5 à 6 maillons.

n peut être égal à O ou à 1
$R_7$ représente :
. un hydrogène
. ou un groupement alkyle linéaire ou ramifié de 1 à 6 atomes de carbone saturé ou insaturé
. ou un groupement benzyle
A-B représente un groupement amide CONH ou rétro amide NHCO ,
lesdits composés se présentant sous forme de mélanges racémiques, sous forme d'énantiomères, de diastéréoisomères ou de stéréoisomères ainsi que leurs mélanges et leurs sels pharmaceutiquement acceptables, ledit procédé étant caractérisé en ce que des composés de formule II:

$R_8NH\text{-}CH\text{-}(R_1)\text{-}CH(R_2)SH$

dans laquelle $R_8$ représente un groupement protecteur de la fonction amine sont condensés sur la dithiodipyridine (III) pour conduire aux composés de formule générale (IV):

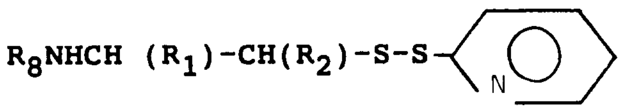

- puis le composé (IV) est condensé sur les composés de formule (V):

$HS\text{-}CH\text{-}(R_3)\text{-}CH(R_4)\text{-}A\text{-}B\text{-}CH(R_5)\text{-}[(CH(R_6)]_n\text{-}COO(R_7)$

pour conduire aux composés de formule (VI):

$R_8NH\text{-}CH(R_1)\text{-}CH(R_2)\text{-}S\text{-}S\text{-}CH(R_3)\text{-}CH(R_4)\text{-}A\text{-}B\text{-}CH(R_5)\text{-}$
$[(CH(R_6)]_n\text{-}COO(R_7)$,
- puis la fonction amine du composé (VI) est déprotégée pour conduire au composé de formule (I).

**2.** Procédé suivant la revendication 1, caractérisé en ce que, si $R_2$ représente un atome d'hydrogène, le produit de formule II est obtenu à partir d'un bêta-amino alcool de formule:

$H_2N\text{-}CH(R_1)\text{-}CH_2OH$ (VII)

- par protection de la fonction amine à l'aide d'un groupe $R_8$ résultant en la formation du produit de formule:

$R_8NH\text{-}CH(R_1)\text{-}CH_2OH$ (VIII)

- puis par activation de la fonction alcool, notamment sous la forme de tosylate, résultant en la formation d'un produit de formule:

$R_8NH\text{-}CH(R_1)\text{-}CH_2OT_S$ (IX)

dans laquelle $T_S$ représente un groupement tosylate,
- puis par transformation du groupement tosylate en groupement acétylthio résultant en la formation du produit de formule:

$R_8NH\text{-}CH(R_1)CH_2\text{-}S\text{-}COCH_3$ (X)

- puis par hydrolyse du produit (X) résultant en la formation du composé de formule (II).

**3.** Procédé selon la revendication 2, caractérisé en ce que si $R_2$ représente un atome d'hydrogène, le produit de formule (X) est obtenu à partir du produit de formule (VIII), en présence de triphényl phosphine et d'un azodicarboxylate de dialkyle.

**4.** Procédé selon l'une des revendications 1 à 3, caractérisé en ce que $R_2$ et $R_3$ représentent tous deux des atomes d'hydrogène, AB représente une liaison amide CONH, $R_1$ représente un groupement $CH_2CH_2S(O)CH_3$ et $R_7$ représente un groupement $CH_2Phe$.

**5.** Procédé selon l'une des revendications 1 à 4, caractérisé en ce que $R_4$ représente

$-CH_2-CH_2S(O)CH_3$.

**6.** Procédé selon l'une des revendications 1 à 4 pour l'obtention du composé de formule :

$H_2N-CH(CH_2CH_2S(O)CH_3)-CH_2-S-S-CH_2-CH(CH_2Phe)-$
$CONHCH_2COOCH_2Phe$.

**7.** Procédé selon l'une des revendications 1 à 4 pour l'obtention du composé répondant à la formule :

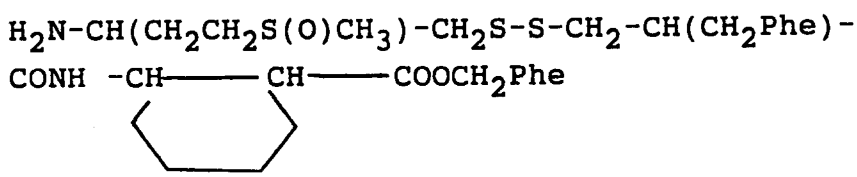

**8.** Procédé selon l'une des revendications 1 à 5 pour l'obtention du composé répondant à la formule:

$H_2N-CH(CH_2CH_2S(O)CH_3)CH_2-S-S-CH_2CH(CH_2CH_2S(O)CH_3)$
$CONHCH(CH_3)COOCH_2Phe$

**9.** Procédé selon l'une des revendications 1 à 5 pour l'obtention du composé répondant à la formule:

$H_2N-CH(CH_2CH_2S(O)CH_3)CH_2-S-S-CH_2-CH(CH_2CH_2S(O)CH_3)-$
$CONH-CH(Phe)-CH_2-COOCH_2Phe$

**10.** Procédé selon l'une des revendications 1 à 5 pour l'obtention du composé répondant à la formule:

$H_2N-CH(CH_2CH_2S(O)CH_3)CH_2-S-S-CH_2-CH(CH_2CH_2S(O)CH_3)-$
$CONH-CH(CH_2Phe)-CH_2-COOCH_2Phe$

**11.** Procédé selon l'une des revendications 1 à 5 pour l'obtention du composé répondant à la formule :

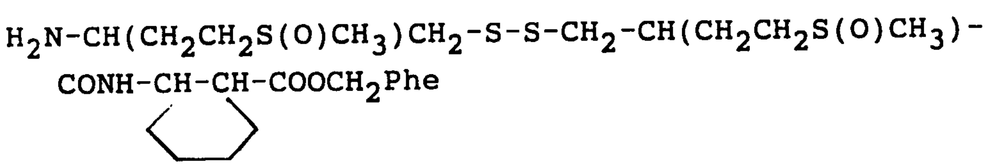

**12.** Procédé selon la revendication 1 pour l'obtention du composé répondant à la formule :

$H_2N-CH(CH_2CH_2SCH_3)CH_2S-S-CH_2CH(CH_2CH(CH_3)_2)-$
$CONHCH(CH_3)COOCH_2Phe$

**13.** Procédé selon la revendication 1 pour l'obtention du composé répondant à la formule

$H_2N-CH(CH_2CH_2SCH_3)CH_2S-S-CH_2CH(CH_2CH_2SCH_3)$
$CONHCH(CH_3)COOCH_2Phe$

**14.** Procédé selon la revendication 1 pour l'obtention du composé répondant à la formule :

$H_2N-CH(CH_2CH(CH_3)_2)-CH_2-S-S-CH_2-CH(CH_2Phe)$
$CONH-CH_2COOCH_2Phe$

**15.** Procédé selon la revendication 1 pour l'obtention du composé répondant à la formule:

$H_2N-CH(CH_2CH(CH_3)_2)-CH_2-S-S-CH_2-CH(CH_2Phe)$
$CONH-CH(CH_3)COOCH_2Phe$

**16.** Procédé selon la revendication 1 pour l'obtention du composé répondant à la formule:

$H_2N-CH(CH_2CH(CH_3)_2)-CH_2-S-S-CH_2-CH(CH_2Phe)$
$CONH-CH(CH_2Phe)COOCH_2Phe$.

**17.** Procédé selon la revendication 1 pour l'obtention du composé répondant à la formule:

$H_2N-CH(CH_2CH_2SCH_3)-CH_2-S-S-CH_2-CH(CH_2Phe)CONH-$
$CH_2-COOCH_2Phe$.

**18.** Procédé selon la revendication 1 pour l'obtention du composé répondant à la formule:

$H_2N-CH(CH_2CH_2SCH_3)-CH_2-S-S-CH_2-CH(CH_2Phe)CONH-$
$CH(CH_3)-COOCH_2Phe$.

**19.** Procédé selon la revendication 1 pour l'obtention du composé répondant à la formule:

$H_2N-CH(CH_2CH_2SCH_3)-CH_2-S-S-CH_2-CH(CH_2Phe)CONH-$
$CH(CH_2Phe)-COOCH_2Phe$.

**20.** Procédé selon la revendication 1 pour l'obtention du composé répondant à la formule:

$H_2N-CH(CH_2CH_2S(O)CH_3)-CH_2-S-S-CH_2-CH(CH_2Phe)$
$CONH-CH(CH_2Phe)COOCH_2Phe$.

**21.** Procédé selon la revendication 1, caractérisé en ce que si $R_2$ représente un groupe $-CH_3$, et si $R_1$ et $R_4$ ont la même signification, le composé de formule (II) est obtenu à partir d'un composé de formule:

$CH_3-CO-SCH(CH_3)CHR_1-CO_2H$ (XI)

- par transformation dudit composé en un produit de formule:

  $CH_3-CO-SCH(CH_3)CHR_1-CON_3$ (XII)

  puis en un produit de formule:

  $CH_3-CO-SCH(CH_3)CHR_1-NHR_8$ (XIII)

- puis par hydrolyse acide du groupement acétylthio.

**22.** Procédé selon la revendication 1, caractérisé en ce que si $R_3$ représente un atome d'hydrogène ou un groupe $CH_3$, et AB un groupement amide CONH, le composé de formule (V) est obtenu à partir d'un composé de formule:

$CH_3-CO-SCH(R_3)CH(R_4)-CO_2H$ (XIV)

- par hydrolyse alcaline dudit composé résultant en la formation du composé de formule:

$(S\ CH(R_3)CH(R_4)\text{-}CO_2H)_2$ (XV)

- puis condensation avec un amino-ester de formule :

$H_2N\text{-}CH(R_5)\text{-}[CH(R_6)]_n\text{-}CO\text{-}OR_7$ (XVI)

résultant en la formation d'un composé de formule:

$(SCHR_3CH(R_4)CONH\ CH(R_5)[CH(R_6)]_4\text{-}COOR_7)_2$ (XVII)

- puis traitement en milieu réducteur.

**23.** Procédé selon la revendication 1, caractérisé en ce que le composé de base de formule XI du procédé selon la revendication 23 peut être obtenu à partir d'un composé de formule:

$(Phe)_3P\text{-}CH(R_1)\text{-}COOCH_3$ (XVIII)

- par condensation avec de l'acétaldéhyde résultant en la formation du composé de formule:

$CH_3CH = C(R_1)\text{-}COOCH_3$ (XIX)

- puis par hydrolyse alcaline résultant en la formation du composé de formule:

$CH_3CH = C(R_1)\text{-}CO_2H$ (XX)

- puis par addition d'acide thioacétique.

**24.** Procédé selon la revendication 1, caractérisé en ce que si $R_3$ représente un atome d'hydrogène ou un groupe $-CH_3$ et si $R_1$ et $R_4$ ont la même signification, et si AB représente un groupe rétro-amide NHCO, le composé de formule (V) est préparé respectivement à partir des composés (X) et (XIII) répondant la formule générale:

$CH_3CO\ S\ CH(R_2)CH(R_1)NHR_8$ (XXI)

- par hydrolyse alcaline résultant en la formation du composé de formule:

$[SCH(R_2)CH(R_1)NHR_8]_2$ (XXII)

- puis par déprotection de la fonction amine conduisant au composé de formule:

$[SCH(R_2)CH(R_1)NH_2]_2$ (XXIII)

- puis par condensation avec des monoacides esters maloniques ou succiniques de formule:

$CO_2H\text{-}CH(R_5)\text{-}[CH(R_6)]_n\text{-}COOR_7$ (XXIV)

résultant en la formation d'un composé de formule:

$[SCH(R_2)CHR_1\text{-}NHCO\text{-}CHR_5\text{-}(CHR_6)_n\text{-}COOR_7]_2$

- puis par traitement en milieu réducteur.

**25.** Procédé de préparation des composés de formule

$H_2N\text{-}CH(R_1)\text{-}CH(R_2)\text{-}S\text{-}S\text{-}CH(R_3)\text{-}CH(R_4)\text{-}A\text{-}B\text{-}Z$ (I)

dans laquelle :
R$_1$ représente :

- une chaîne alkylée hydrocarbonée ou insaturée linéaire ou ramifiée comportant de 1 à 6 atomes de carbone, substituée ou non par un groupe OR dans laquelle R est un hydrogène, une chaîne hydrocarbonée linéaire ou ramifiée de $C_1$ à $C_4$, ou un reste phényle ou benzyle; un groupe thioéther SR, R ayant la même définition que ci-dessus ou un groupe thioéther oxydé S(O)R, R ayant la même définition que ci-dessus,
- un groupe méthyl cycloalkyle à 5 à 6 atomes de carbone, un groupe benzyl ou phényl, éventuellement substitué:
  . par 1 à 5 atomes d'halogène notamment de fluor,
  . ou par un groupe OR' ou SR', R' étant un groupement alkylé de 1 à 4 atomes de carbone, le thioéther SR' étant oxydé ou non sur l'atome de soufre,
  . ou par un groupe amino éventuellement mono ou disubstitué par un groupe alkyle aliphatique de 1 à 6 atomes de carbone, oxydé ou non sur la fonction amine,
- un groupe méthylène substitué par un hétérocycle à 5 ou 6 atomes de carbone, aromatique ou saturé, possédant comme hétéroatome, un atome d'azote, un atome d'oxygène ou un atome de soufre, les atomes d'azote et de soufre étant éventuellement oxydés sous forme de N-oxyde ou de S-oxyde.

$R_2$ représente indépendamment, un atome d'hydrogène ou un groupement méthyle
$R_3$ représente un atome d'hydrogène ou une chaîne alkyle linéaire ou ramifiée
$R_4$ représente :

- une chaîne hydrocarbonée saturée ou insaturée, linéaire ou ramifiée comportant de 1 à 6 atomes de carbone, substitué ou non par un groupe hydroxy, éther OR dans lequel le groupe R a la même signification que précédemment, un groupe thiol SH, thioéther SR ( R ayant la même signification que précédemment) ou un groupe thioéther oxydé en sulfoxyde,
- ou un groupe méthylcycloalkyle de 5 à 6 atomes de carbone, un groupe benzyl ou phényl, éventuellement substitué par 1 à 5 atomes d'halogène notamment de fluor, par un hydroxyle ou un thiol, un éther ou un thioéther OR'ou SR', R' ayant de 1 à 4 atomes de carbone, le thioéther étant oxydé ou non sur l'atome de soufre, par un groupe amino éventuellement mono ou disubstitué par un groupe alkyle aliphatique de 1 à 6 atomes de carbone, oxydé ou non sur la fonction amine,
- ou un groupe méthylène substitué par un hétérocycle à 5 ou 6 atomes de carbone, aromatique ou saturé, possédant comme hétéroatome, un atome d'azote, un atome d'oxygène ou un atome de soufre, les atomes d'azote et de soufre étant éventuellement oxydés sous forme de N-oxyde ou de S-oxyde,

  le groupement Z représente :
  * un groupement morpholinyle $-N-C_4H_8O$
  * un groupement pipéridinyle $-NC_5H_{10}$, éventuellement substitué par un reste -OH, un reste $-CO_2H$, un reste $COOR^1$, dans lequel $R^1$ représente une chaîne alkyle linéaire ou ramifiée contenant 1 à 6 atomes de carbone, un reste phényle ou un reste benzyle
  * un groupement pyralozinyle $-N-C_4H_8-N-R^2$ dans lequel $R^2$ représente une chaîne alkylée contenant de 1 à 6 atomes de carbone, un reste benzyle ou un reste phényle,

A-B représente un groupement amide CONH ou rétro amide NHCO ,
lesdits composés se présentant sous formes de mélanges racémiques, sous formes d'énantiomères, de diastéréoisomères ou de stéréoisomères ainsi que leurs mélanges et leurs sels pharmaceutiquement acceptables, ledit procédé étant caractérisé en ce que des composés de formule :

$(SCH(R_3)CH(R_4)-CO_2H)_2$ (XV)

sont condensés avec une hydrazine de formule

$H_2N-Z$ (XXV)

résultant en la formation d'un composé de formule:

$(SCH(R_3)CH(R_4)-CONH-Z)_2$ (XXVI)

qui est condensé sur un composé de formule:

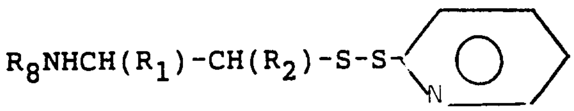 (IV)

pour conduire au composé de formule (I).

**26.** Procédé selon la revendication 25 pour l'obtention d'un composé répondant à la formule:

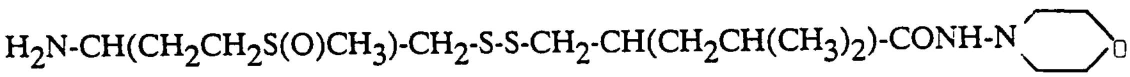

**27.** Procédé de préparation d'une composition pharmaceutique, caractérisée en ce qu'on associe au moins un composé obtenu par un procédé selon l'une quelconque des revendications 1 à 26, avec un ou plusieurs diluants ou adjuvants compatibles ou pharmaceutiquement acceptables.

**28.** Procédé selon la revendication 27, caractérisé en ce que la composition est destinée au traitement de la dépression et de la douleur.

**29.** Procédé selon l'une des revendications 27 et 28, caractérisé en ce que la composition est destinée à l'administration intraveineuse, per os, sous-cutanée ou intrapéritonéale.

**Claims**

**Claims for the following Contracting States : AT, BE, CH, DE, DK, FR, GB, IT, LI, LU, NL, SE**

**1.** Chemical compounds corresponding to the formula :

$H_2N-CH(R_1)-CH(R_2)-S-S-CH(R_3)-CH(R_4)-A-B-Z$ (I)

in which :

$R_1$ represents:

- a saturated or unsaturated, linear or branched hydrocarbonated alkyl chain containing from 1 to 6 carbon atoms, unsubstituted or substituted with a group OR in which R is a hydrogen, a linear or branched C1 to C4 hydrocarbon chain or a phenyl or benzyl residue; a thioether group SR, R having the same definition as above, or an oxidized thioether group S(O)R, R having the same definition as above,
- a methylcycloalkyl group containing 5 to 6 carbon atoms, a benzyl or phenyl group, optionally substituted:
  - * with 1 to 5 halogen, in particular fluorine, atoms,
  - • or with a group OR' or SR', R' being an alkyl group having 1 to 4 carbon atoms, the thioether SR' being oxidized or otherwise on the sulfur atom,
  - • or with an amino group optionally mono- or disubstituted with an aliphatic alkyl group having 1 to 6 carbon atoms, oxidized or otherwise on the amine function,
- a methylene group substituted with an aromatic or saturated heterocycle containing 5 or 6 carbon atoms, possessing as the hetero atom a nitrogen atom, an oxygen atom or a sulfur atom, the nitrogen and sulfur atoms optionally being oxidized in the form of N-oxide or S-oxide.

$R_2$ independently represents a hydrogen atom or a methyl group.

$R_3$ represents a hydrogen atom or a linear or branched alkyl chain.

$R_4$ represents:

- a saturated or unsaturated, linear or branched hydrocarbon chain containing from 1 to 6 carbon atoms, unsubstituted or substituted with a hydroxyl group, an ether group OR in which the group R has the same meaning as above, a thiol SH, thioether SR group (R having the same meaning as above) or a thioether group oxidized to sulfoxide,

- or a methylcycloalkyl group having 5 to 6 carbon atoms, a benzyl or phenyl group, optionally substituted with 1 to 5 halogen, in particular fluorine, atoms, with a hydroxyl or a thiol, an ether or a thioether OR' or SR', R' having from 1 to 4 carbon atoms, the thioether being oxidized or otherwise on the sulfur atom, with an amino group optionally mono- or disubstituted with an aliphatic alkyl group having 1 to 6 carbon atoms, oxidized or otherwise on the amine function,
- or a methylene group substituted with an aromatic or saturated heterocycle containing 5 or 6 carbon atoms, possessing as the hetero atom a nitrogen atom, an oxygen atom or a sulfur atom, the nitrogen and sulfur atoms optionally being oxidized in the form of N-oxide or S-oxide.

Z can represent:

* a group $-CH(R_5)-[CH(R_6)]_n-COO(R_7)$

in which

$R_5$ and $R_6$ can represent, independently of one another:

- a hydrogen,
- or a linear or branched alkyl group having 1 to 6 carbon atoms, unsubstituted or substituted with hydroxyl or ether OR, thiol SH or thioether SR groups (R having the definition specified above),
- or a phenyl (Phe) or benzyl group unsubstituted or substituted with 1 to 5 halogen atoms; a group OR' or SR', R' having the definition specified above.

$R_5$ and $R_6$ can also each represent saturated or unsaturated hydrocarbon chains containing from 1 to 6 carbon atoms in which 1 to 2 carbon atoms may be substituted by an oxygen, sulfur or nitrogen atom, capable of leading to the formation of one or more saturated or aromatic 5- to 6-membered hydrocarbon rings or saturated or aromatic 5- to 6-membered heterocyclic rings.

n can be equal to 0 or 1.

$R_7$ represents:

- a hydrogen,
- or a saturated or unsaturated, linear or branched alkyl group having 1 to 6 carbon atoms,
- or a benzyl group.

* a morpholinyl group $-N-C_4H_8O$

* a piperidyl group $-N-C_5H_{10}$ optionally substituted with an -OH residue, a $-CO_2H$ residue, a residue $COOR^1$ in which $R^1$ represents a linear or branched alkyl chain containing 1 to 6 carbon atoms, a phenyl residue or a benzyl residue

* a pyralozinyl group $-N-C_4H_8-NH$, a substituted pyralozinyl group $-NC_4H_8-N-R^2$ in which $R^2$ represents an alkyl chain containing from 1 to 6 carbon atoms, a benzyl residue or a phenyl residue.

A-B represents an amide CONH or retro-amide NHCO group said compounds being in the forms of racemic mixtures or in the forms of enantiomers, of diastereoisomers or of stereoisomers, as well as mixtures thereof and their pharmaceutically acceptable salts.

**2.** Compounds according to claim 1, characterized in that Z represents a group

$-CH(R_5)-[CH(R_6)]_n-COO(R_7)$,

$R_2$ and $R_3$ both represent hydrogen atoms, AB represents an amide link CONH, $R_1$ represents a $-CH_2CH_2S(O)CH_3$ group, and $R_7$ represents a $-CH_2Phe$ group.

**3.** Compounds according to claim 2, characterized in that $R_4$ represents $-CH_2-CH_2S(O)CH_3$.

**4.** Compound according to claim 2, corresponding to the formula: $H_2N-CH(CH_2CH_2S(O)CH_3)-CH_2S-S-CH_2-CH(CH_2Phe)CONHCH_2COOCH_2Phe$

**5.** Compound according to claim 2, corresponding to the formula:

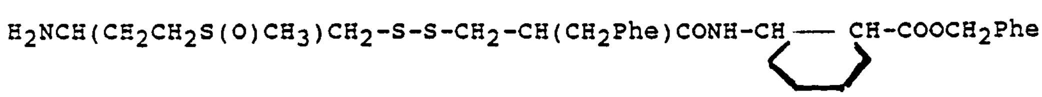

**6.** Compound according to claim 3, corresponding to the formula:

$H_2N\text{-}CH(CH_2CH_2S(O)CH_3)CH_2\text{-}S\text{-}S\text{-}CH_2CH(CH_2CH_2S(O)CH_3)CONHCH(CH_3)COOCH_2Phe$

**7.** Compound according to claim 3, corresponding to the formula:

$H_2N\text{-}CH(CH_2CH_2S(O)CH_3)\text{-}CH_2\text{-}S\text{-}S\text{-}CH_2\text{-}CH(CH_2CH_2S(O)CH_3)CONHCH(Phe)\text{-}CH_2\text{-}COOCH_2Phe$

**8.** Compound according to claim 3, corresponding to the formula:

$H_2N\text{-}CH(CH_2CH_2S(O)CH_3)\text{-}CH_2\text{-}S\text{-}S\text{-}CH_2\text{-}CH(CH_2CH_2S(O)CH_3)CONHCH(CH_2Phe)\text{-}CH_2COOCH_2Phe$

**9.** Compound according to claim 3, corresponding to the formula:

$H_2N\text{-}CH(CH_2CH_2S(O)CH_3)CH_2\text{-}S\text{-}S\text{-}CH_2\text{-}CH(CH_2CH_2S(O)CH_3)CONH\text{-}CH\text{-}CH\text{-}$

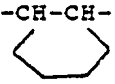

$COOCH_2Phe$

**10.** Compound according to claim 1, corresponding to the formula:

$H_2NCH(CH_2CH_2SCH_3)CH_2S\text{-}S\text{-}CH_2CH(CH_2CH(CH_3)_2)CONHCH(CH_3)COOCH_2Phe$

**11.** Compound according to claim 1, corresponding to the formula:

$H_2NCH(CH_2CH_2SCH_3)CH_2\text{-}S\text{-}S\text{-}CH_2\text{-}CH(CH_2CH_2SCH_3)CONHCH(CH_3)COOCH_2Phe$

**12.** Compound according to claim 1, corresponding to the formula:

$H_2N\text{-}CH(CH_2CH(CH_3)_2)\text{-}CH_2\text{-}S\text{-}S\text{-}CH_2\text{-}CH(CH_2Phe)CONH\text{-}CH_2COOCH_2Phe$

**13.** Compound according to claim 1, corresponding to the formula:

$H_2N\text{-}CH(CH_2CH(CH_3)_2)\text{-}CH_2\text{-}S\text{-}S\text{-}CH_2\text{-}CH(CH_2Phe)CONH\text{-}CH(CH_3)COOCH_2Phe$.

**14.** Compound according to claim 1, corresponding to the formula:

$H_2N\text{-}CH(CH_2CH(CH_3)_2)\text{-}CH_2\text{-}S\text{-}S\text{-}CH_2\text{-}CH(CH_2Phe)CONH\text{-}CH(CH_2Phe)COOCH_2Phe$.

**15.** Compound according to claim 1, corresponding to the formula:

$H_2N\text{-}CH(CH_2CH_2SCH_3)\text{-}CH_2\text{-}S\text{-}S\text{-}CH_2\text{-}CH(CH_2Phe)CONH\text{-}CH_2\text{-}COOCH_2Phe$.

**16.** Compound according to claim 1, corresponding to the formula:

$H_2N\text{-}CH(CH_2CH_2SCH_3)\text{-}CH_2\text{-}S\text{-}S\text{-}CH_2\text{-}CH(CH_2Phe)CONH\text{-}CH(CH_3)COOCH_2Phe$.

**17.** Compound according to claim 1, corresponding to the formula :

$H_2N\text{-}CH(CH_2CH_2SCH_3)CH_2\text{-}S\text{-}S\text{-}CH_2\text{-}CH(CH_2Phe)CONH\text{-}CH(CH_2Phe)COOCH_2Phe$.

**18.** Compound according to claim 1, corresponding to the formula :

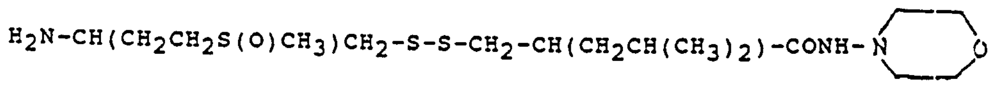

**19.** Compound according to claim 1, corresponding to the formula :

$H_2N-CH(CH_2CH_2S(O)CH_3)-CH_2-S-S-CH_2-CH(CH_2Phe)-CONH-CH(CH_2Phe)$
$COOCH_2Phe$.

**20.** Method for preparing the compounds of formula I in which Z represents a group

$-CH(R_5)-[CH(R_6)]_n-COO(R_7)$

characterized in that compounds of formula II

$R8NH-CH(R_1)-CH(R_2)SH$

in which $R_8$ represents a group protecting the amine function, are condensed with dithiodipyridine (III) to yield the compounds of general formula (IV):

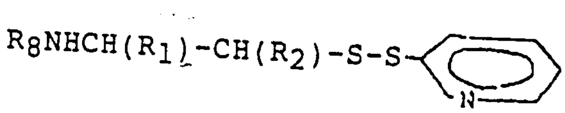

- the compound (IV) is then condensed with the compounds of formula (V):

  $HS-CH(R_3)-CH(R_4)-A-B-CH(R_5)-[CH(R_6)]_n-COO(R_7)$

  to yield the compounds of formula (VI):

  $R_8NH-CH(R_1)-CH(R_2)-S-S-CH(R_3)-CH(R_4)-A-B-CH(R_5)-[(CH(R_6)]_n-COO(R_7)$

- the amine function of the compound (VI) is then deprotected to yield the compound of formula (I).

**21.** Method according to claim 20, characterized in that, if $R_2$ represents a hydrogen atom, the product of formula II is obtained from a beta-amino alcohol of formula:

$H_2N-CH(R_1)-CH_2OH$ (VII)

- by protection of the amine function using a group $R_8$, resulting in the formation of the product of formula:

  $R_8NH-CH(R_1)-CH_2OH$ (VIII)

- then by activation of the alcohol function, in particular in the form of tosylate, resulting in the formation of a product of formula:

  $R_8NH-CH(R_1)\ CH_2OT_S$ (IX)

  in which $T_S$ represents a tosylate group,
- then by conversion of the tosylate group to an acetylthio group, resulting in the formation of the product of formula:

$R_8NH-CH(R_1)-CH_2-S-COCH_3$ (X)

- then by hydrolysis of the product (X), resulting in the formation of the compound of formula (II).

**22.** Method according to claim 20, characterized in that, if $R_2$ represents a hydrogen atom, the product of formula (X) is obtained from the product of formula (VIII) in the presence of triphenylphosphine and a dialkyl azodicarboxylate.

**23.** Method according to claim 20, characterized in that, if $R_2$ represents a $-CH_3$ group, and if $R_1$ and $R_4$ have the same meaning, the compound of formula (II) is obtained from a compound of formula:

$CH_3-CO-SCH(CH_3)CHR_1-CO_2H$ (XI)

- by conversion of said compound to a product of formula:

  $CH_3-CO-SCH(CH_3)CHR_1-CON_3$ (XII)

- then to a product of formula:

  $CH_3-CO-SCH(CH_3)CHR_1-NHR_8$ (XIII)

- then by acid hydrolysis of the acetylthio group.

**24.** Method according to claim 20, characterized in that, if $R_3$ represents a hydrogen atom or a $CH_3$ group, and AB an amide group CONH, the compound of formula (V) is obtained from a compound of formula:

$CH_3-CO-SCH(R_3)CH(R_4)-CO_2H$ (XIV)

- by alkaline hydrolysis of said compound, resulting in the formation of the compound of formula:

  $(S\ CH(R_3)CH(R_4)-CO_2H)_2$ (XV)

- followed by condensation with an amino ester of formula:

  $H_2N-CH(R_5)-[CH(R_6)]_n-CO-OR_7$ (XVI)

  resulting in the formation of a compound of formula:

  $(S\ CHR_3CH(R_4)CONH\ CH(R_5)[CH(R_6)]_4-COOR_7)_2$ (XVII)

- followed by treatment in a reducing medium.

**25.** Method according to claim 20, characterized in that the starting compound of formula XI of the method according to claim 23 may be obtained from a compound of formula:

$(Phe)_3P-CH(R_1)-COOCH_3$ (XVIII)

- by condensation with acetaldehyde, resulting in the formation of the compound of formula:

  $CH_3CH = C(R_1)-COOCH_3$ (XIX)

- then by alkaline hydrolysis, resulting in the formation of the compound of formula:

  $CH_3CH=C(R_1)-CO_2H$ (XX)

- then by addition of thioacetic acid.

**26.** Method according to claim 20, characterized in that, if $R_3$ represents a hydrogen atom or a $-CH_3$ group and if $R_1$ and $R_4$ have the same meaning, and if AB represents a retro-amide group NHCO, the compound of formula (V) is prepared, respectively, from the compounds (X) and (XIII) corresponding to the general formula:

$CH_3CO\ S\ CH(R_2)CH(R_1)NHR_8$ (XXI)

- by alkaline hydrolysis, resulting in the formation of the compound of formula:

  $[SCH(R_2)CH(R_1)NHR_8]_2$ (XXII)

- then by deprotection of the amine function, yielding the compound of formula:

  $[SCH(R_2)CH(R_1)NH_2]_2$ (XXIII)

- then by condensation with malonic or succinic acid monoesters of formula:

  $CO_2H\text{-}CH(R_5)\text{-}[CH(R_6)]_n\text{-}COOR_7$ (XXIV)

  resulting in the formation of a compound of formula:

  $[SCH(R_2)CHR_1\text{-}NHCO\text{-}CHR_5\text{-}(CHR_6)_n\text{-}COOR_7]_2$

- then by treatment in a reducing medium.

**27.** Method for preparing the compounds of formula I in which the group Z represents:
  * a morpholinyl group $-N\text{-}C_4H_8O$
  * a piperidyl group $-N\ C_5H_{10}$ optionally substituted with an -OH residue, a $-CO_2H$ residue, a residue $COOR^1$ in which $R^1$ represents a linear or branched alkyl chain containing 1 to 6 carbon atoms, a phenyl residue or a benzyl residue
  * a pyralozinyl group $-NC_4H_8\text{-}N\text{-}R^2$ in which $R^2$ represents an alkyl chain containing from 1 to 6 carbon atoms, a benzyl residue or a phenyl residue,

characterized in that compounds of formula

$(SCH(R_3)CH(R_4)\text{-}CO_2H)_2$ (XV)

are condensed with a hydrazine of formula

$H_2N\text{-}Z$ (XXV)

resulting in the formation of a compound of formula

$(SCH(R_3)CH(R_4)\text{-}CONH\text{-}Z)_2$ (XXVI)

which is condensed with a compound of formula

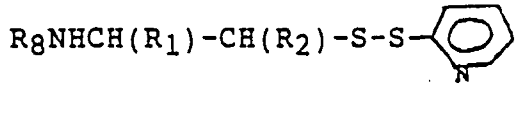

(IV)

to yield the compound of formula (I).

**28.** Pharmaceutical composition, characterized in that it contains at least one compound according to one of claims 1 to 19, or obtained by a method according to any one of claims 20 to 27, in combination with one or more compatible or pharmaceutically acceptable diluents or adjuvants.

**29.** Pharmaceutical composition according to claim 28, characterized in that it is intended for the treatment of depression and pain.

**30.** Composition according to one of claims 28 and 29 characterized in that it is intended for the intravenous, oral, subcutaneous or intraperitoneal administration routes.

**31.** Use of compounds according to one of claims 1 to 19 or obtained by a process according to one of claims 2O to 27 for making a medecine for the treatment of depression.

**32.** Use of compounds according to one of claims 1 to 19 or obtained by a process according to one of claims 2O to 27 for making a medecine for the treatment of pain.

**Claims for the following Contracting State : ES**

**1.** Method for preparing the compounds of formula I in

$H_2N\text{-}CH(R_1)\text{-}CH(R_2)\text{-}S\text{-}S\text{-}CH(R_3)\text{-}CH(R_4)\text{-}A\text{-}B\text{-}Z$ (I)

in which :
$R_1$ represents:
- a saturated or unsaturated, linear or branched hydrocarbonated alkyl chain containing from 1 to 6 carbon atoms, unsubstituted or substituted with a group OR in which R is a hydrogen, a linear or branched C1 to C4 hydrocarbon chain or a phenyl or benzyl residue; a thioether group SR, R having the same definition as above, or an oxidized thioether group S(O)R, R having the same definition as above,
- a methylcycloalkyl group containing 5 to 6 carbon atoms, a benzyl or phenyl group, optionally substituted:
  * with 1 to 5 halogen, in particular fluorine, atoms,
  * or with a group OR' or SR', R' being an alkyl group having 1 to 4 carbon atoms, the thioether SR' being oxidized or otherwise on the sulfur atom,
  * or with an amino group optionally mono- or disubstituted with an aliphatic alkyl group having 1 to 6 carbon atoms, oxidized or otherwise on the amine function,
- a methylene group substituted with an aromatic or saturated heterocycle containing 5 or 6 carbon atoms, possessing as the hetero atom a nitrogen atom, an oxygen atom or a sulfur atom, the nitrogen and sulfur atoms optionally being oxidized in the form of N-oxide or S-oxide.

$R_2$ independently represents a hydrogen atom or a methyl group.
$R_3$ represents a hydrogen atom or a linear or branched alkyl chain.
$R_4$ represents:
- a saturated or unsaturated, linear or branched hydrocarbon chain containing from 1 to 6 carbon atoms, unsubstituted or substituted with a hydroxyl group, an ether group OR in which the group R has the same meaning as above, a thiol SH, thioether SR group (R having the same meaning as above) or a thioether group oxidized to sulfoxide,
- or a methylcycloalkyl group having 5 to 6 carbon atoms, a benzyl or phenyl group, optionally substituted with 1 to 5 halogen, in particular fluorine, atoms, with a hydroxyl or a thiol, an ether or a thioether OR' or SR', R' having from 1 to 4 carbon atoms, the thioether being oxidized or otherwise on the sulfur atom, with an amino group optionally mono- or disubstituted with an aliphatic alkyl group having 1 to 6 carbon atoms, oxidized or otherwise on the amine function,
- or a methylene group substituted with an aromatic or saturated heterocycle containing 5 or 6 carbon atoms, possessing as the hetero atom a nitrogen atom, an oxygen atom or a sulfur atom, the nitrogen and sulfur atoms optionally being oxidized in the form of N-oxide or S-oxide,
  which Z represents a group

  $\text{-}CH(R_5)\text{-}[CH(R_6)]_n\text{-}COO(R_7)$

in which

$R_5$ and $R_6$ can represent, independently of one another:

* a hydrogen,
* a linear or branched alkyl group having 1 to 6 carbon atoms, unsubstituted or substituted with hydroxyl or ether OR, thiol SH or thioether SR groups (R having the definition specified above),

- or a phenyl (Phe) or benzyl group unsubstituted or substituted with 1 to 5 halogen atoms; a group OR' or SR', R' having the definition specified above.

$R_5$ and $R_6$ can also each represent saturated or unsaturated hydrocarbon chains containing from 1 to 6 carbon atoms in which 1 to 2 carbon atoms may be substituted by an oxygen, sulfur or nitrogen atom, capable of leading to the formation of one or more saturated or aromatic 5- to 6-membered hydrocarbon rings or saturated or aromatic 5- to 6- membered heterocyclic rings.

n can be equal to O or 1.

$R_7$ represents:

* a hydrogen,
* or a saturated or unsaturated, linear or branched alkyl group having 1 to 6 carbon atoms,
* or a benzyl group.

A-B represents an amide CONH or retro-amide NHCO group said compounds being in the forms of racemic mixtures or in the forms of enantiomers, of diastereoisomers or of stereoisomers, as well as mixtures thereof and their pharmaceutically acceptable salts,

characterized in that compounds of formula II

$R_8NH-CH(R_1)-CH(R_2)SH$

in which $R_8$ represents a group protecting the amine function, are condensed with dithiodipyridine (III) to yield the compounds of general formula (IV):

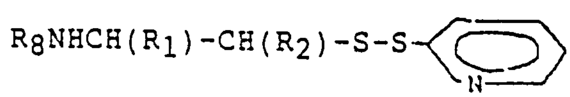

- the compound (IV) is then condensed with the compounds of formula (V):

  $HS-CH(R_3)-CH(R_4)-A-B-CH(R_5)-[CH(R_6)]_n-COO(R_7)$

  to yield the compounds of formula (VI):

  $R_8NH-CH(R_1)-CH(R_2)-S-S-CH(R_3)-CH(R_4)-A-B-CH(R_5)-[(CH(R_6)]_n-COO(R_7)$

- the amine function of the compound (VI) is then deprotected to yield the compound of formula (I).

**2.** Method according to claim 1, characterized in that, if $R_2$ represents a hydrogen atom, the product of formula II is obtained from a beta-amino alcohol of formula :

$H_2N-CH(R_1)-CH_2OH$ (VII)

- by protection of the amine function using a group $R_8$, resulting in the formation of the product of formula:

  $R_8NH-CH(R_1)-CH_2OH$ (VIII)

- then by activation of the alcohol function, in particular in the form of tosylate, resulting in the formation of a product of formula:

  $R_8NH-CH(R_1)CH_2OT_S$ (IX)

  in which $T_S$ represents a tosylate group,

- then by conversion of the tosylate group to an acetylthio group, resulting in the formation of the product of formula :

$R_8NH\text{-}CH(R_1)\text{-}CH_2\text{-}S\text{-}COCH_3$ (X)

- then by hydrolysis of the product (X), resulting in the formation of the compound of formula (II).

**3.** Method according to claim 2, characterized in that, if $R_2$ represents a hydrogen atom, the product of formula (X) is obtained from the product of formula (VIII) in the presence of triphenylphosphine and a dialkyl azodicarboxylate.

**4.** Method according to one of claims 1 to 3, characterized in that Z represents a group

$-CH(R_5)\text{-}[CH(R_6)]_n\text{-}COO(R_7)$,

$R_2$ and $R_3$ both represent hydrogen atoms, AB represents an amide link CONH, $R_1$ represents a $-CH_2CH_2S(O)CH_3$ group, and $R_7$ represents a $-CH_2Phe$ group.

**5.** Method according to one of claims 1 to 4, characterized in that $R_4$ represents $-CH_2\text{-}CH_2S(O)CH_3$.

**6.** Method according to one of claims 1 to 4, for the obtention of the compound corresponding to the formula:

$H_2N\text{-}CH(CH_2CH_2S(O)CH_3)\text{-}CH_2S\text{-}S\text{-}CH_2\text{-}CH(CH_2Phe)CONHCH_2COOCH_2Phe$

**7.** Method according to one of claims 1 to 4, for the obtention of the compound corresponding to the formula:

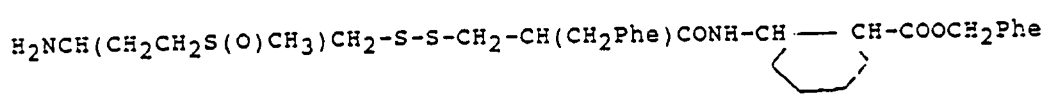

**8.** Method according to one of claims 1 to 4, for the obtention of the compound corresponding to the formula :

$H_2N\text{-}CH(CH_2CH_2S(O)CH_3)CH_2\text{-}S\text{-}S\text{-}CH_2CH(CH_2CH_2S(O)CH_3)CONHCH(CH_3)$
$COOCH_2Phe$

**9.** Method according to one of claims 1 to 4, for the obtention of the compound , corresponding to the formula:

$H_2N\text{-}CH(CH_2CH_2S(O)CH_3)\text{-}CH_2\text{-}S\text{-}S\text{-}CH_2\text{-}CH(CH_2CH_2S(O)CH_3)CONHCH(Phe)\text{-}$
$CH_2\text{-}COOCH_2Phe$

**10.** Method according to one of claims 1 to 4, for the obtention of the compound corresponding to the formula:

$H_2N\text{-}CH(CH_2CH_2S(O)CH_3)\text{-}CH_2\text{-}S\text{-}S\text{-}CH_2\text{-}CH(CH_2CH_2S(O)CH_3)CONHCH(CH_2Phe)\text{-}$
$CH_2COOCH_2Phe$

**11.** Method according to one of claims 1 to 4, for the obtention of the compound corresponding to the formula:

$H_2N-CH(CH_2CH_2S(O)CH_3)CH_2-S-S-CH_2-CH(CH_2CH_2S(O)CH_3)CONH-CH-CH-$ 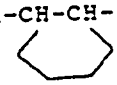

$COOCH_2Phe$ .

**12.** Method according to claim 1, for the obtention of the compound corresponding to the formula:

$H_2NCH(CH_2CH_2SCH_3)CH_2S\text{-}S\text{-}CH_2CH(CH_2CH(CH_3)_2)CONHCH(CH_3)COOCH_2Phe$

**13.** Method according to claim 1 for the obtention of the compound corresponding to the formula:

$H_2NCH(CH_2CH_2SCH_3)CH_2\text{-}S\text{-}S\text{-}CH_2\text{-}CH(CH_2CH_2SCH_3)CONHCH(CH_3)COOCH_2Phe$

**14.** Method according to claim 1 for the obtention of the compound corresponding to the formula:

$H_2N\text{-}CH(CH_2CH(CH_3)_2)\text{-}CH_2\text{-}S\text{-}S\text{-}CH_2\text{-}CH(CH_2Phe)CONH\text{-}CH_2COOCH_2Phe$

**15.** Method according to claim 1 for the obtention of the compound corresponding to the formula:

$H_2N\text{-}CH(CH_2CH(CH_3)_2)\text{-}CH_2\text{-}S\text{-}S\text{-}CH_2\text{-}CH(CH_2Phe)CONH\text{-}CH(CH_3)COOCH_2Phe$.

**16.** Method according to claim 1, for the obtention of the compound corresponding to the formula:

$H_2N\text{-}CH(CH_2CH(CH_3)_2)\text{-}CH_2\text{-}S\text{-}S\text{-}CH_2\text{-}CH(CH_2Phe)CONH\text{-}CH(CH_2Phe)$
$COOCH_2Phe$.

**17.** Method according to claim 1, for the obtention of the compound corresponding to the formula:

$H_2N\text{-}CH(CH_2CH_2SCH_3)\text{-}CH_2\text{-}S\text{-}S\text{-}CH_2\text{-}CH(CH_2Phe)CONH\text{-}CH_2\text{-}COOCH_2Phe$.

**18.** Method according to claim 1, for the obtention of the compound corresponding to the formula:

$H_2N\text{-}CH(CH_2CH_2SCH_3)\text{-}CH_2\text{-}S\text{-}S\text{-}CH_2\text{-}CH(CH_2Phe)CONH\text{-}CH(CH_3)COOCH_2Phe$.

**19.** Method according to claim 1, for the obtention of the compound corresponding to the formula:

$H_2N\text{-}CH(CH_2CH_2SCH_3)CH_2\text{-}S\text{-}S\text{-}CH_2\text{-}CH(CH_2Phe)CONH\text{-}CH(CH_2Phe)COOCH_2Phe$.

**20.** Method according to claim 1, for the obtention of the compound corresponding to the formula:

$H_2N\text{-}CH(CH_2CH_2S(O)CH_3)\text{-}CH_2\text{-}S\text{-}S\text{-}CH_2\text{-}CH(CH_2Phe)\text{-}CONH\text{-}CH(CH_2Phe)$
$COOCH_2Phe$.

**21.** Method according to claim 1, characterized in that, if $R_2$ represents a $-CH_3$ group, and if $R_1$ and $R_4$ have the same meaning, the compound of formula (II) is obtained from a compound of formula:

$CH_3\text{-}CO\text{-}SCH(CH_3)CHR_1\text{-}CO_2H$ (XI)

- by conversion of said compound to a product of formula:

  $CH_3\text{-}CO\text{-}SCH\text{-}(CH_3)CHR_1\text{-}CON_3$ (XII)

- then to a product of formula:

  $CH_3\text{-}CO\text{-}SCH(CH_3)CHR_1\text{-}NHR_8$ (XIII)

- then by acid hydrolysis of the acetylthio group.

**22.** Method according to claim 1, characterized in that, if $R_3$ represents a hydrogen atom or a $CH_3$ group, and AB an amide group CONH, the compound of formula (V) is obtained from a compound of formula:

$CH_3$-CO-SCH($R_3$)CH($R_4$)-$CO_2$H (XIV)

- by alkaline hydrolysis of said compound, resulting in the formation of the compound of formula:

  [SCH($R_3$)CH($R_4$)-$CO_2$H$]_2$ (XV)

- followed by condensation with an amino ester of formula:

  $H_2$N-CH($R_5$)-[CH($R_6$)$]_n$-CO-O$R_7$ (XVI)

  resulting in the formation of a compound of formula:

  [SCH$R_3$CH($R_4$)CONH CH($R_5$)[CH($R_6$)$]_4$-COO$R_7$$]_2$ (XVII)

- followed by treatment in a reducing medium.

**23.** Method according to claim 1, characterized in that the starting compound of formula XI of the method according to claim 23 may be obtained from a compound of formula:

(Phe$)_3$P-CH($R_1$)-COO$CH_3$ (XVIII)

- by condensation with acetaldehyde, resulting in the formation of the compound of formula:

  $CH_3$CH = C($R_1$)-COO$CH_3$ (XIX)

- then by alkaline hydrolysis, resulting in the formation of the compound of formula:

  $CH_3$CH = C($R_1$)-$CO_2$H (XX)

- then by addition of thioacetic acid.

**24.** Method according to claim 1, characterized in that, if $R_3$ represents a hydrogen atom or a -$CH_3$ group and if $R_1$ and $R_4$ have the same meaning, and if AB represents a retro-amide group NHCO, the compound of formula (V) is prepared, respectively, from the compounds (X) and (XIII) corresponding to the general formula:

$CH_3$CO S CH($R_2$)CH($R_1$)NH$R_8$ (XXI)

- by alkaline hydrolysis, resulting in the formation of the compound of formula:

  [SCH($R_2$)CH($R_1$)NH$R_8$$]_2$ (XXII)

- then by deprotection of the amine function, yielding the compound of formula:

  [SCH($R_2$)CH($R_1$)$NH_2$$]_2$ (XXIII)

- then by condensation with malonic or succinic acid monoesters of formula:

  $CO_2$H-CH($R_5$)-[CH($R_6$)$]_n$-COO$R_7$ (XXIV)

  resulting in the formation of a compound of formula:

  [SCH($R_2$)CH$R_1$-NHCO-CH$R_5$-(CH$R_6$$)_n$-COO$R_7$$]_2$

- then by treatment in a reducing medium.

**25.** Method for preparing the compounds of formula I

$H_2N-CH(R_1)-CH(R_2)-S-S-CH(R_3)-CH(R_4)-A-B-Z$ (I)

in which :
R1 represents:

- a hydrocarbonated or unsaturated, linear or branched alkyl chain containing from 1 to 6 carbon atoms, unsubstituted or substituted with a group OR in which R is a hydrogen, a linear or branched C1 to C4 hydrocarbon chain or a phenyl or benzyl residue; a thioether group SR, R having the same definition as above, or an oxidized thioether group S(O)R, R having the same definition as above,
- a methylcycloalkyl group containing 5 to 6 carbon atoms, a benzyl or phenyl group, optionally substituted:
  - * with 1 to 5 halogen, in particular fluorine, atoms,
  - * or with a group OR' or SR', R' being an alkyl group having 1 to 4 carbon atoms, the thioether SR' being oxidized or otherwise on the sulfur atom,
  - * or with an amino group optionally mono- or disubstituted with an aliphatic alkyl group having 1 to 6 carbon atoms, oxidized or otherwise on the amine function,
- a methylene group substituted with an aromatic or saturated heterocycle containing 5 or 6 carbon atoms, possessing as the hetero atom a nitrogen atom, an oxygen atom or a sulfur atom, the nitrogen and sulfur atoms optionally being oxidized in the form of N-oxide or S-oxide.

$R_2$ independently represents a hydrogen atom or a methyl group.
$R_3$ represents a hydrogen atom or a linear or branched alkyl chain.
$R_4$ represents:

- a saturated or unsaturated, linear or branched hydrocarbon chain containing from 1 to 6 carbon atoms, unsubstituted or substituted with a hydroxyl group, an ether group OR in which the group R has the same meaning as above, a thiol SH, thioether SR group (R having the same meaning as above) or a thioether group oxidized to sulfoxide,
- or a methylcycloalkyl group having 5 to 6 carbon atoms, a benzyl or phenyl group, optionally substituted with 1 to 5 halogen, in particular fluorine, atoms, with a hydroxyl or a thiol, an ether or a thioether OR' or SR', R' having from 1 to 4 carbon atoms, the thioether being oxidized or otherwise on the sulfur atom, with an amino group optionally mono- or disubstituted with an aliphatic alkyl group having 1 to 6 carbon atoms, oxidized or otherwise on the amine function,
- or a methylene group substituted with an aromatic or saturated heterocycle containing 5 or 6 carbon atoms, possessing as the hetero atom a nitrogen atom, an oxygen atom or a sulfur atom, the nitrogen and sulfur atoms optionally being oxidized in the form of N-oxide or S-oxide,

  in which the group Z represents:
  - * a morpholinyl group $-N-C_4H_8O$
  - * a piperidyl group $-N\ C_5H_{10}$ optionally substituted with an -OH residue, a $-CO_2H$ residue, a residue $COOR^1$ in which $R^1$ represents a linear or branched alkyl chain containing 1 to 6 carbon atoms, a phenyl residue or a benzyl residue,
  - * a pyralozinyl group $-NC_4H_8-N-R^2$ in which $R^2$ represents an alkyl chain containing from 1 to 6 carbon atoms, a benzyl residue or a phenyl residue,

    in which
    $R_5$ and $R_6$ can represent, independently of one another:
  - * a hydrogen,
  - * a linear or branched alkyl group having 1 to 6 carbon atoms, unsubstituted or substituted with hydroxyl or ether OR, thiol SH or thioether SR groups (R having the definition specified above),
- or a phenyl (Phe) or benzyl group unsubstituted or substituted with 1 to 5 halogen atoms; a group OR' or SR', R' having the definition specified above,

  $R_5$ and $R_6$ can also each represent saturated or unsaturated hydrocarbon chains containing from 1 to 6 carbon atoms in which 1 to 2 carbon atoms may be substituted by an oxygen, sulfur or nitrogen atom, capable of leading to the formation of one or more saturated or aromatic 5- to 6-membered hydrocarbon rings or saturated or aromatic 5- to 6- membered heterocyclic rings.

n can be equal to O or 1.
$R_7$ represents:

* a hydrogen,
* or a saturated or unsaturated, linear or branched alkyl group having 1 to 6 carbon atoms,
* or a benzyl group

A-B represents an amide CONH or retro-amide NHCO group said compounds being in the forms of racemic mixtures or in the forms of enantiomers, of diastereoisomers or of stereoisomers, as well as mixtures thereof and their pharmaceutically acceptable salts,
characterized in that compounds of formula

$[SCH(R_3)CH(R_4)\text{-}CO_2H]_2$ (XV)

are condensed with a hydrazine of formula

$H_2N\text{-}Z$ (XXV)

resulting in the formation of a compound of formula

$[SCH(R_3)CH(R_4)\text{-}CONH\text{-}Z]_2$ (XXVI)

which is condensed with a compound of formula

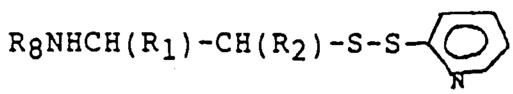

(IV)

to yield the compound of formula (I).

**26.** Method according to claim 25, for the obtention of the compound corresponding to the formula:

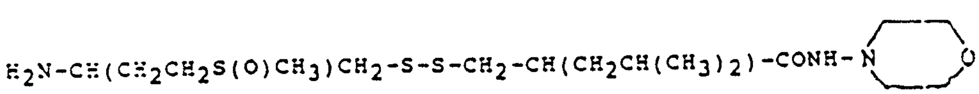

**27.** Process for the preparation of a pharmaceutical composition, characterized in that at least one compound obtained by a method according to any one of claims 1 to 26 is associated with one or more compatible or pharmaceutically acceptable diluents or adjuvants.

**28.** Process according to claim 27 , characterized in that the composition is intended for the treatment of depression and pain.

**29.** Method according to one of claims 27 and 28, characterized in that the composition is intended to the intravenous, oral, subcutaneous or intraperitoneal administration routes.

**Patentansprüche**
**Patentansprüche für folgende Vertragsstaaten : AT, BE, CH, DE, DK, FR, GB, IT, LI, LU, NL, SE**

**1.** Chemische Verbindungen mit der Formel:

$H_2N\text{-}CH(R_1)\text{-}CH(R_2)\text{-}S\text{-}S\text{-}CH(R_3)\text{-}CH(R_4)\text{-}A\text{-}B\text{-}Z$ (I),

in der:
$R_1$ bedeutet:

- eine gesättigte oder ungesättigte, lineare oder verzweigte Alkylkohlenwasserstoffkette mit 1 bis 6 Kohlenstoffatomen, die nicht substituiert oder substituiert ist durch eine OR-Gruppe, in der R ein Wasserstoffatom, eine lineare oder verzweigte $C_1$- bis $C_4$-Kohlenwasserstoffketteoder ein Phenyl- oder Benzylrest ist, eine Thioethergruppe SR, wobei R wie oben definiert ist, oder eine oxidierte Thioethergruppe S(O)R, wobei R wie oben definiert ist;
- eine Methylcycloalkylgruppe mit 5 bis 6 Kohlenstoffatomen, eine Benzyl- oder Phenylgruppe, die gegebenenfalls substituiert sind durch:
  . 1 bis 5 Halogen-, insbesondere Fluoratome,
  . oder eine OR'- oder SR'-Gruppe, wobei R' eine Alkylgruppe mit 1 bis 4 Kohlenstoffatomen ist und der Thioether SR' am Schwefelatom oxidiert oder nicht oxidiert ist,
  . oder eine Aminogruppe, die gegebenenfalls durch eine aliphatische Alkylgruppe mit 1 bis 6 Kohlenstoffatomen mono- oder disubstituiert ist und die an der Aminofunktion oxidiert oder nicht oxidiert ist;
- eine Methylengruppe, die durch einen aromatischen oder gesättigten Heterocyclus mit 5 oder 6 Kohlenstoffatomen substituiert ist, der als Heteroatom ein Stickstoffatom, ein Sauerstoffatom oder ein Schwefelatom besitzt, wobei das Stickstoff- bzw. das Schwefelatom gegebenenfalls in Form eines N-Oxids bzw. S-Oxids oxidiert vorliegen;

$R_2$ bedeutet unabhängig ein Wasserstoffatom oder eine Methylgruppe;
$R_3$ bedeutet ein Wasserstoffatom oder eine lineare oder verzweigte Alkylkette;
$R_4$ bedeutet:

- eine gesättigte oder ungesättigte, lineare oder verzweigte Kohlenwasserstoffkette mit 1 bis 6 Kohlenstoffatomen, die nicht substituiert oder substituiert ist durch eine Hydroxygruppe, eine Ethergruppe OR, in der die Gruppe R das gleiche wie vorher bedeutet, eine Thiolgruppe SH, eine Thioethergruppe SR (wobei R das gleiche wie vorher bedeutet) oder eine zum Sulfoxid oxidierte Thioethergruppe;
- oder eine Methylcycloalkylgruppe mit 5 bis 6 Kohlenstoffatomen, eine Benzyl- oder Phenylgruppe, gegebenenfalls substituiert durch 1 bis 5 Halogen-, insbesondere Fluoratome, eine Hydroxy- oder eine Thiolgruppe, eine Ether- oder Thioethergruppe OR' bzw. SR', wobei R' 1 bis 4 Kohlenstoffatome besitzt und der Thioether am Schwefelatom oxidiert oder nicht oxidiert ist, eine Aminogruppe, die gegebenenfalls durch eine aliphatische Alkylgruppe mit 1 bis 6 Kohlenstoffatomen mono- oder disubstituiert ist und die an der Aminofunktion oxidiert oder nicht oxidiert ist;
- oder eine Methylengruppe, die durch einen aromatischen oder gesättigten Heterocyclus mit 5 oder 6 Kohlenstoffatomen substituiert ist, der als Heteroatom ein Stickstoffatom, ein Sauerstoffatom oder ein Schwefelatom besitzt, wobei das Stickstoff- bzw. das Schwefelatom gegebenenfalls in Form eines N-Oxids bzw. S-Oxids oxidiert vorliegen;

  Z kann bedeuten:
- eine $-CH(R_5)-[CH(R_6)]_n-COO(R_7)$-Gruppe, in der $R_5$ und $R_6$ voneinander unabhängig bedeuten können:
  . ein Wasserstoffatom,
  . oder eine lineare oder verzweigte Alkylgruppe mit 1 bis 6 Kohlenstoffatomen, die nicht substituiert oder substituiert ist durch Hydroxy- oder Ether(OR), Thiol- (SH) oder Thioethergruppen (SR, wobei R wie oben definiert ist),
  . oder eine Phenyl- (Phe) oder Benzylgruppe, die nicht substituiert oder substituiert ist durch 1 bis 5 Halogenatome, eine OR'- oder SR'-Gruppe, wobei R' wie oben definiert ist;

$R_5$ und $R_6$ können auch jede gesättigte oder ungesättigte Kohlenwasserstoffkette mit 1 bis 6 Kohlenstoffatomen bedeuten, in der 1 bis 2 Kohlenstoffatome mit einem Sauerstoff-, Schwefel- oder Stickstoffatom substituiert sein können, die zur Bildung eines oder mehrerer gesättigter oder aromatischer 5- bis 6-gliedriger Kohlenwasserstoffringe oder gesättigter oder aromatischer 5- bis 6-gliedriger Heterocyclen führen können;
n kann gleich 0 oder 1 sein;
$R_7$ bedeutet:

. ein Wasserstoffatom
. oder eine gesättigte oder ungesättigte lineare oder verzweigte Alkylgruppe mit 1 bis 6 Kohlenstoffatomen
. oder eine Benzylgruppe;

  - eine Morpholingruppe $-N(C_4H_8O)$
  - eine Piperidingruppe $-N(C_5H_{10})$, die gegebenenfalls durch einen Rest -OH, einen Rest $-CO_2H$ oder einen Rest $COOR^1$ substituiert ist, wobei $R^1$ eine lineare oder verzweigte Alkylgruppe mit

1 bis 6 Kohlenstoffatomen, einen Phenyl- oder einen Benzylrest darstellt;

- eine Pyrazolingruppe $-N(C_4H_8)NH$, eine substituierte Pyrazolingruppe $-N(C_4H_8)N-R^2$, wobei $R^2$ eine Alkylkette mit 1 bis 6 Kohlenstoffatomen, einen Benzylrest oder einen Phenylrest darstellt;

A-B bedeutet eine Amidgruppe CONH oder eine umgekehrte Amidgruppe NHCO;
wobei die Verbindungen in Form racemischer Gemische, in Form von Enantiomeren, Diastereomeren oder Stereoisomeren sowie deren Gemischen sowie ihren pharmazeutisch akzeptablen Salzen vorliegen.

**2.** Verbindungen gemäß Anspruch 1, dadurch gekennzeichnet, daß Z eine $CH(R_5)-[CH(R_6)]_nCOO(R_7)$-Gruppe ist und $R_2$ und $R_3$ beides Wasserstoffatome sind, AB eine Amidbindung CONH ist, $R_1$ eine $CH_2CH_2S(O)CH_3$-Gruppe ist und $R_7$ eine $CH_2Phe$-Gruppe ist.

**3.** Verbindungen gemäß Anspruch 2, dadurch gekennzeichnet, daß $R_4$ $-CH_2-CH_2S(O)CH_3$ ist.

**4.** Verbindung gemäß Anspruch 2 mit der Formel:

$H_2N-CH(CH_2CH_2S(O)CH_3)-CH_2S-S-CH_2-CH(CH_2Phe)CONHCH_2COOCH_2Phe.$

**5.** Verbindung gemäß Anspruch 2 mit der Formel:

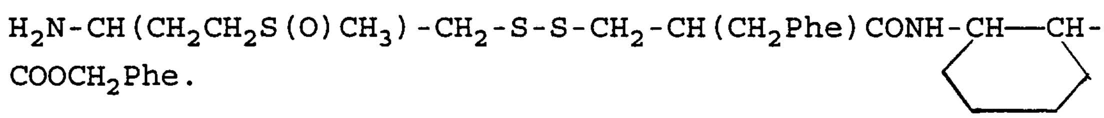

**6.** Verbindung gemäß Anspruch 3 mit der Formel:

$H_2N-CH(CH_2CH_2S(O)CH_3)CH_2-S-S-CH_2CH(CH_2CH_2S(O)CH_3)CONH-CH(CH_3)COOCH_2Phe.$

**7.** Verbindung gemäß Anspruch 3 mit der Formel:

$H_2N-CH(CH_2CH_2S(O)CH_3)-CH_2-S-S-CH_2-CH(CH_2CH_2S(O)CH_3)CONH-CH(Phe)-CH_2-COOCH_2Phe.$

**8.** Verbindung gemäß Anspruch 3 mit der Formel:

$H_2N-CH(CH_2CH_2S(O)CH_3)-CH_2-S-S-CH_2-CH(CH_2CH_2S(O)CH_3)CONH-CH(CH_2Phe)-CH_2-COOCH_2Phe.$

**9.** Verbindung gemäß Anspruch 3 mit der Formel:

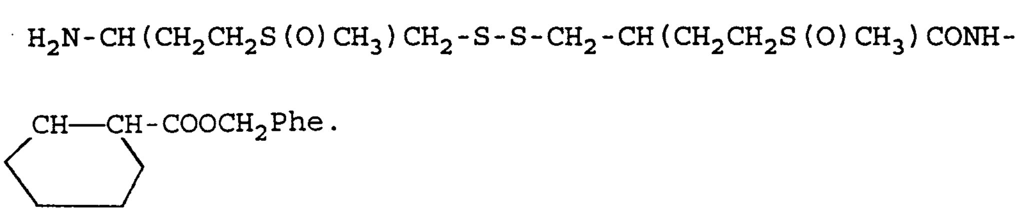

**10.** Verbindung gemäß Anspruch 1 mit der Formel:

$H_2N-CH(CH_2CH_2SCH_3)CH_2S-S-CH_2CH(CH_2CH(CH_3)_2)CONHCH(CH_3)COO CH_2Phe.$

**11.** Verbindung gemäß Anspruch 1 mit der Formel:

$H_2N-CH(CH_2CH_2SCH_3)CH_2-S-S-CH_2-CH(CH_2CH_2SCH_3)CONHCH(CH_3)COO$
$CH_2Phe$.

**12.** Verbindung gemäß Anspruch 1 mit der Formel:

$H_2N-CH(CH_2CH(CH_3)_2)-CH_2-S-S-CH_2-CH(CH_2Phe)CONH-CH_2COOCH_2Phe$.

**13.** Verbindung gemäß Anspruch 1 mit der Formel:

$H_2N-CH(CH_2CH(CH_3)_2)-CH_2-S-S-CH_2-CH(CH_2Phe)CONH-CH_2(CH_3)COO$
$CH_2Phe$.

**14.** Verbindung gemäß Anspruch 1 mit der Formel:

$H_2N-CH(CH_2CH(CH_3)_2)-CH_2-S-S-CH_2-CH(CH_2Phe)CONH-CH_2(CH_2Phe)$-
$COOCH_2Phe$.

**15.** Verbindung gemäß Anspruch 1 mit der Formel:

$H_2N-CH(CH_2CH_2SCH_3)-CH_2-S-S-CH_2-CH(CH_2Phe)CONH-CH_2-COOCH_2Phe$.

**16.** Verbindung gemäß Anspruch 1 mit der Formel:

$H_2N-CH(CH_2CH_2SCH_3)-CH_2-S-S-CH_2-CH(CH_2Phe)CONH-CH(CH_3)-COO$
$CH_2Phe$.

**17.** Verbindung gemäß Anspruch 1 mit der Formel:

$H_2N-CH(CH_2CH_2SCH_3)CH_2-S-S-CH_2-CH(CH_2Phe)CONH-CH(CH_2Phe)-COO$
$CH_2Phe$.

**18.** Verbindung gemäß Anspruch 1 mit der Formel:

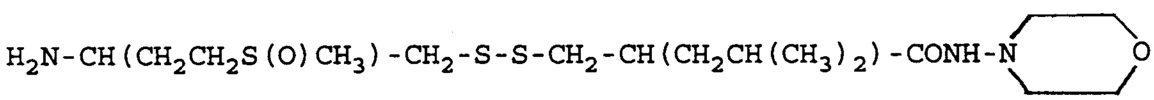

**19.** Verbindung gemäß Anspruch 1 mit der Formel:

$H_2N-CH(CH_2CH_2S(O)CH_3)-CH_2-S-S-CH_2-CH(CH_2Phe)-CONH-CH(CH_2$
$Phe)COOCH_2Phe$.

**20.** Verfahren zur Herstellung von Verbindungen der Formel I, in der Z eine $CH(R_5)-[CH(R_6)]_n-COO(R_7)$-Gruppe darstellt, dadurch gekennzeichnet, daß Verbindungen mit der Formel II:

$R_8NH-CH(R_1)-CH(R_2)SH$,

in der $R_8$ eine Schutzgruppe für die Aminfunktion darstellt, mit Dithiodipyridin (III) kondensiert werden, um zu den Verbindungen der allgemeinen Formel (IV) zu gelangen:

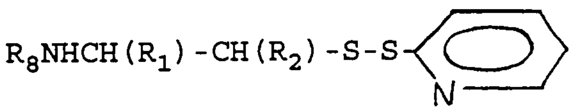

- dann wird Verbindung (IV) mit den Verbindungen der Formel (V) kondensiert: $HS\text{-}CH(R_3)\text{-}CH(R_4)\text{-}A\text{-}B\text{-}CH(R_5)\text{-}[(CH(R_6)]_n\text{-}COO(R_7)$, um zu den Verbindungen der Formel (VI) zu gelangen:

  $R_8NH\text{-}CH(R_1)\text{-}CH(R_2)\text{-}S\text{-}S\text{-}CH(R_3)\text{-}CH(R_4)\text{-}A\text{-}B\text{-}CH(R_5)\text{-}[(CH(R_6)]_n\text{-}COO(R_7)$

  anschließend wird die Schutzgruppe von der Aminfunktion der Verbindung (VI) entfernt, um zur Verbindung der Formel (I) zu gelangen.

**21.** Verfahren gemäß Anspruch 20, dadurch gekennzeichnet, daß, wenn $R_2$ ein Wasserstoffatom ist, das Produkt mit der Formel II erhalten wird, indem man von einem $\beta$-Aminoalkohol ausgeht mit der Formel:

$H_2N\text{-}CH(R_1)\text{-}CH_2OH$ (VII)

- durch Schutz der Aminfunktion mit Hilfe einer Gruppe $R_8$, was zur Bildung des Produkts mit der folgenden Formel führt:

  $R_8NH\text{-}CH(R_1)\text{-}CH_2OH$ (VIII),

- durch anschließende Aktivierung der Alkoholfunktion, insbesondere in Form eines Tosylats, was zur Bildung eines Produkts mit der folgenden Formel führt:

  $R_8NH\text{-}CH(R_1)\text{-}CH_2OTs$ (IX),

  wobei Ts eine Tosylatgruppe bedeutet,
- durch anschließende Umwandlung der Tosylatgruppe in eine Acetylthiogruppe, was zur Bildung des Produkts mit der folgenden Formel führt:

  $R_8NH\text{-}CH(R_1)\text{-}CH_2\text{-}S\text{-}COCH_3$ (X),

- durch anschließende Hydrolyse des Produkts (X), was zur Bildung der Verbindung mit der Formel (II) führt.

**22.** Verfahren gemäß Anspruch 20, dadurch gekennzeichnet, daß, wenn $R_2$ ein Wasserstoffatom ist, das Produkt mit der Formel (X) erhalten wird, indem man vom Produkt mit der Formel (VIII) ausgeht, in Gegenwart von Triphenylphosphin und einem Dialkylazodicarboxylat.

**23.** Verfahren gemäß Anspruch 20, dadurch gekennzeichnet, daß, wenn $R_2$ eine $CH_3$-Gruppe ist und wenn $R_1$ und $R_4$ gleich sind, die Verbindung mit der Formel (II) erhalten wird, indem man von einer Verbindung ausgeht mit der Formel:

$CH_3\text{-}CO\text{-}SCH(CH_3)CHR_1\text{-}CO_2H$ (XI),

- durch Umsetzung dieser Verbindung zu einem Produkt mit der Formel:

  $CH_3\text{-}CO\text{-}SCH(CH_3)CHR_1\text{-}CON_3$ (XII),

  anschließend zu einem Produkt mit der Formel:

  $CH_3\text{-}CO\text{-}SCH(CH_3)CHR_1\text{-}NHR_8$ (XIII),

durch anschließende saure Hydrolyse der Acetylthiogruppe.

**24.** Verfahren gemäß Anspruch 20, dadurch gekennzeichnet, daß, wenn $R_3$ ein Wasserstoffatom oder eine $CH_3$-Gruppe ist und wenn AB eine Amidgruppe CONH ist, die Verbindung mit der Formel (V) erhalten wird, indem man von einer Verbindung ausgeht mit der Formel:

$CH_3$-CO-SCH($R_3$)CH($R_4$)-$CO_2$H (XIV),

- durch alkalische Hydrolyse dieser Verbindung, was zur Bildung der Verbindung mit der folgenden Formel führt:

  (SCH($R_3$)CH($R_4$)-$CO_2$H)$_2$ (XV),

- durch anschließende Kondensation mit einem Aminoester der Formel:

  $H_2$N-CH($R_5$)-[CH($R_6$)]$_n$-CO-O$R_7$ (XVI),

  was zur Bildung einer Verbindung mit der folgenden Formel führt:

  (SCH$R_3$CH($R_4$)CONH-CH($R_5$)[CH($R_6$)]$_n$-COO$R_7$)$_2$ (XVII),

- anschließende Behandlung in reduzierendem Milieu.

**25.** Verfahren gemäß Anspruch 20, dadurch gekennzeichnet, daß die Ausgangsverbindung mit der Formel XI des Verfahrens gemäß Anspruch 23 erhalten werden kann, indem man von einer Verbindung ausgeht mit der Formel:

(Phe)$_3$P-CH($R_1$)-COO$CH_3$ (XVIII),

- durch Kondensation mit Acetaldehyd, was zur Bildung der Verbindung mit der folgenden Formel führt:

  $CH_3$CH = C($R_1$)-COO$CH_3$ (XIX),

- durch anschließende alkalische Hydrolyse, was zur Bildung der Verbindung mit der folgenden Formel führt:

  $CH_3$CH = C($R_1$)-$CO_2$H (XX),

- durch anschließendes Zufügen von Thioessigsäure.

**26.** Verfahren gemäß Anspruch 20, dadurch gekennzeichnet, daß, wenn $R_3$ ein Wasserstoffatom oder eine $CH_3$-Gruppe ist und wenn $R_1$ und $R_4$ gleich sind und wenn AB eine umgekehrte Amidgruppe NHCO ist, die Verbindung mit der Formel (V) hergestellt wird, indem man von den Verbindungen (X) bzw. (XIII) ausgeht, denen die folgende allgemeine Formel zukommt:

$CH_3$CO-S-CH($R_2$)CH($R_1$)NH$R_8$ (XXI),

- durch alkalische Hydrolyse, was zur Bildung der Verbindung mit der folgenden Formel führt:

  [SCH($R_2$)CH($R_1$)NH$R_8$]$_2$ (XXII),

- durch anschließende Entfernung der Schutzgruppe von der Aminofunktion, was zur Verbindung mit der folgenden Formel führt:

  [SCH($R_2$)CH($R_1$)$NH_2$]$_2$ (XXIII),

- durch anschließende Kondensation mit Malonsäure- oder Bernsteinsäure-Monoestern mit der Formel:

$CO_2H\text{-}CH(R_5)\text{-}[CH(R_6)]_n\text{-}COOR_7$ (XXIV),

was zur Bildung einer Verbindung mit der folgenden Formel führt:

$[SCH(R_2)CHR_1\text{-}NHCO\text{-}CHR_5\text{-}(CHR_6)_n\text{-}COOR_7]_2$,

- durch anschließende Behandlung in reduzierendem Milieu.

**27.** Verfahren zur Herstellung von Verbindungen mit der Formel I, in der die Gruppe Z bedeutet:
- eine Morpholingruppe $-N(C_4H_8O)$
- eine Piperidingruppe $-N(C_5H_{10})$, die gegebenenfalls durch einen Rest -OH, einen Rest $-CO_2H$ oder einen Rest $COOR^1$ substituiert ist, wobei $R^1$ eine lineare oder verzweigte Alkylgruppe mit 1 bis 6 Kohlenstoffatomen, einen Phenyl- oder einen Benzylrest darstellt;
- eine Pyrazolingruppe $-N(C_4H_8)N\text{-}R^2$, wobei $R^2$ eine Alkylkette mit 1 bis 6 Kohlenstoffatomen, einen Benzylrest oder einen Phenylrest darstellt,

dadurch gekennzeichnet, daß Verbindungen mit der Formel:

$(SCH(R_3)CH(R_4)\text{-}CO_2H)_2$ (XV)

mit einem Hydrazin der Formel

$H_2N\text{-}Z$ (XXV)

kondensiert werden, was zur Bildung einer Verbindung mit

der folgenden Formel führt:

$(SCH(R_3)CH(R_4)\text{-}CONH\text{-}Z)_2$ (XXVI),

welche mit einer Verbindung der Formel

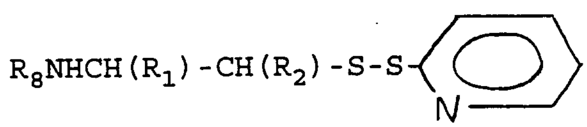

(IV)

kondensiert wird, was zur Verbindung mit der Formel (I) führt.

**28.** Pharmazeutische Zusammensetzung, dadurch gekennzeichnet, daß sie wenigstens eine Verbindung gemäß einem der Ansprüche 1 bis 19 oder eine, die nach einem Verfahren gemäß einem der Ansprüche 20 bis 27 erhalten wurde, in Verbindung mit einem oder mehreren vertraglichen oder pharmazeutisch akzeptablen Verdünnungsmitteln oder Adjuvantien enthält.

**29.** Pharmazeutische Zusammensetzung gemäß Anspruch 28, dadurch gekennzeichnet, daß sie zur Behandlung von Depressionen und von Schmerzen bestimmt ist.

**30.** Zusammensetzung gemäß einem der Ansprüche 28 und 29, die zur intravenösen, subcutanen, intraperitonealen oder zur oralen Verabreichung bestimmt ist.

**31.** Verwendung der Verbindungen gemäß einem der Ansprüche 1 bis 19 bzw. solcher, die nach einem Verfahren gemäß einem der Ansprüche 20 bis 27 erhalten wurden, zur Herstellung eines Medikaments zur Behandlung von Depressionen.

32. Verwendung der Verbindungen gemäß einem der Ansprüche 1 bis 19 bzw. solcher, die nach einem Verfahren gemäß einem der Ansprüche 20 bis 27 erhalten wurden, zur Herstellung eines Medikaments zur Behandlung von Schmerzen.

**Patentansprüche für folgenden Vertragsstaat : ES**

1. Verfahren zur Herstellung von Verbindungen der Formel I:

$H_2N\text{-}CH(R_1)\text{-}CH(R_2)\text{-}S\text{-}S\text{-}CH(R_3)\text{-}CH(R_4)\text{-}A\text{-}B\text{-}Z$ (I),

in der:
$R_1$ bedeutet:
- eine gesättigte oder ungesättigte, lineare oder verzweigte Alkylkohlenwasserstoffkette mit 1 bis 6 Kohlenstoffatomen, die nicht substituiert oder substituiert ist durch eine OR-Gruppe, in der R ein Wasserstoffatom, eine lineare oder verzweigte $C_1$- bis $C_4$-Kohlenwasserstoffketteoder ein Phenyl- oder Benzylrest ist, eine Thioethergruppe SR, wobei R wie oben definiert ist, oder eine oxidierte Thioethergruppe S(O)R, wobei R wie oben definiert ist;
- eine Methylcycloalkylgruppe mit 5 bis 6 Kohlenstoffatomen, eine Benzyl- oder Phenylgruppe, die gegebenenfalls substituiert sind durch:
  . 1 bis 5 Halogen-, insbesondere Fluoratome,
  . oder eine OR'- oder SR'-Gruppe, wobei R' eine Alkylgruppe mit 1 bis 4 Kohlenstoffatomen ist und der Thioether SR' am Schwefelatom oxidiert oder nicht oxidiert ist,
  . oder eine Aminogruppe, die gegebenenfalls durch eine aliphatische Alkylgruppe mit 1 bis 6 Kohlenstoffatomen mono- oder disubstituiert ist und die an der Aminofunktion oxidiert oder nicht oxidiert ist;
- eine Methylengruppe, die durch einen aromatischen oder gesättigten Heterocyclus mit 5 oder 6 Kohlenstoffatomen substituiert ist, der als Heteroatom ein Stickstoffatom, ein Sauerstoffatom oder ein Schwefelatom besitzt, wobei das Stickstoff- bzw. das Schwefelatom gegebenenfalls in Form eines N-Oxids bzw. S-Oxids oxidiert vorliegt ;

$R_2$ bedeutet unabhängig ein Wasserstoffatom oder eine Methylgruppe;
$R_3$ bedeutet ein Wasserstoffatom oder eine lineare oder verzweigte Alkylkette;
$R_4$ bedeutet:
- eine gesättigte oder ungesättigte, lineare oder verzweigte Kohlenwasserstoffkette mit 1 bis 6 Kohlenstoffatomen, die nicht substituiert oder substituiert ist durch eine Hydroxygruppe, eine, Ethergruppe OR, in der die Gruppe R das gleiche wie vorher bedeutet, eine Thiolgruppe SH, eine Thioethergruppe SR (wobei R das gleiche wie vorher bedeutet) oder eine zum Sulfoxid oxidierte Thioethergruppe;
- oder eine Methylcycloalkylgruppe mit 5 bis 6 Kohlenstoffatomen, eine Benzyl- oder Phenylgruppe, gegebenenfalls substituiert durch 1 bis 5 Halogen-, insbesondere Fluoratome, eine Hydroxy- oder eine Thiolgruppe, eine Ether- oder Thioethergruppe OR' bzw. SR', wobei R' 1 bis 4 Kohlenstoffatome besitzt und der Thioether am Schwefelatom oxidiert oder nicht oxidiert ist, eine Aminogruppe, die gegebenenfalls durch eine aliphatische Alkylgruppe mit 1 bis 6 Kohlenstoffatomen mono- oder disubstituiert ist und die an der Aminofunktion oxidiert oder nicht oxidiert ist;
- oder eine Methylengruppe, die durch einen aromatischen oder gesättigten Heterocyclus mit 5 oder 6 Kohlenstoffatomen substituiert ist, der als Heteroatom ein Stickstoffatom, ein Sauerstoffatom oder ein Schwefelatom besitzt, wobei das Stickstoff- bzw. das Schwefelatom gegebenenfalls in Form eines N-Oxids bzw. S-Oxids oxidiert vorliegen;
  Z bedeutet:
- eine $\text{-}CH(R_5)\text{-}[CH(R_6)]_n\text{-}COO(R_7)$-Gruppe, in der $R_5$ und $R_6$ voneinander unabhängig bedeuten können:
  . ein Wasserstoffatom,
  . oder eine lineare oder verzweigte Alkylgruppe mit 1 bis 6 Kohlenstoffatomen, die nicht substituiert oder substituiert ist durch Hydroxy- oder Ether-(OR), Thiol- (SH) oder Thioethergruppen (SR, wobei R wie oben definiert ist),
  . oder eine Phenyl- (Phe) oder Benzylgruppe, die nicht substituiert oder substituiert ist durch 1 bis 5 Halogenatome, eine OR'- oder SR'-Gruppe, wobei R' wie oben definiert ist;

$R_5$ und $R_6$ können auch jede gesättigte oder ungesättigte Kohlenwasserstoffkette mit 1 bis 6 Kohlenstoffatomen bedeuten, in der 1 bis 2 Kohlenstoffatome mit einem Sauerstoff-, Schwefel- oder

Stickstoffatom substituiert sein können, die zur Bildung eines oder mehrerer gesättigter oder aromatischer 5- bis 6-gliedriger Kohlenwasserstoffringe oder gesättigter oder aromatischer 5- bis 6-gliedriger Heterocyclen führen können;
n kann gleich 0 oder 1 sein;
$R_7$ bedeutet:

- . ein Wasserstoffatom
- . oder eine gesättigte oder ungesättigte lineare oder verzweigte Alkylgruppe mit 1 bis 6 Kohlenstoffatomen
- . oder eine Benzylgruppe;

A-B bedeutet eine Amidgruppe CONH oder eine umgekehrte Amidgruppe NHCO;
wobei die Verbindungen in Form racemischer Gemische, in Form von Enantiomeren, Diastereomeren oder Stereoisomeren sowie deren Gemischen sowie ihren pharmazeutisch akzeptablen Salzen vorliegen, dadurch gekennzeichnet, daß Verbindungen mit der Formel II:

$R_8NH-CH(R_1)-CH(R_2)SH$,

wobei $R_8$ eine Schutzgruppe für die Aminofunktion bedeutet, mit Dithiodipyridin kondensiert werden, um zu den Verbindungen mit der allgemeinen Formel (IV) zu gelangen:

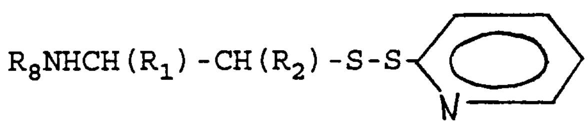

- anschließend wird die Verbindung (IV) mit Verbindungen der Formel (V) kondensiert:
  $HS-CH(R_3)-CH(R_4)-A-B-CH(R_5)-[(CH(R_6)]_n-COO(R_7)$, um zu den Verbindungen der Formel (VI) zu gelangen:

  $R_8NH-CH(R_1)-CH(R_2)-S-S-CH(R_3)-CH(R_4)-A-B-CH(R_5)-[(CH(R_6)]_n-$
  $COO(R_7)$,

  anschließend wird die Schutzgruppe von der Aminfunktion der Verbindung (VI) entfernt, um zur Verbindung der Formel (I) zu gelangen.

**2.** Verfahren gemäß Anspruch 1, dadurch gekennzeichnet, daß, wenn $R_2$ ein Wasserstoffatom ist, das Produkt mit der Formel II erhalten wird, indem man von einem $\beta$-Aminoalkohol ausgeht mit der Formel:

$H_2N-CH(R_1)-CH_2OH$ (VII)

- durch Schutz der Aminofunktion mit Hilfe einer Gruppe $R_8$, was zur Bildung des Produkts mit der folgenden Formel führt:

  $R_8NH-CH(R_1)-CH_2OH$ (VIII),

- durch anschließende Aktivierung der Alkoholfunktion, insbesondere in Form eines Tosylats, was zur Bildung eines Produkts mit der folgenden Formel führt:

  $R_8NH-CH(R_1)-CH_2OTs$ (IX),

  wobei Ts eine Tosylatgruppe bedeutet,
- durch anschließende Umwandlung der Tosylatgruppe in eine Acetylthiogruppe, was zur Bildung des Produkts mit der folgenden Formel führt:

  $R_8NH-CH(R_1)-CH_2-S-COCH_3$ (X),

- durch anschließende Hydrolyse des Produkts (X), was zur Bildung der Verbindung mit der Formel (II) führt.

**3.** Verfahren gemäß Anspruch 2, dadurch gekennzeichnet, daß, wenn $R_2$ ein Wasserstoffatom ist, das Produkt mit der Formel (X) erhalten wird, indem man vom Produkt mit der Formel (VIII) ausgeht, in Gegenwart von Triphenylphosphin und einem Dialkylazodicarboxylat.

**4.** Verfahren gemäß einem der Ansprüche 1 bis 3, dadurch gekennzeichnet, daß $R_2$ und $R_3$ beides Wasserstoffatome sind, AB eine Amidbindung CONH ist, $R_1$ eine $CH_2CH_2S(O)CH_3$-Gruppe ist und $R_7$ eine $CH_2Phe$-Gruppe ist.

**5.** Verfahren gemäß einem der Ansprüche 1 bis 4, dadurch gekennzeichnet, daß $R_4$

$-CH_2-CH_2S(O)CH_3$

ist.

**6.** Verfahren gemäß einem der Ansprüche 1 bis 4 zur Erhaltung der Verbindung mit der Formel:

$H_2N-CH(CH_2CH_2S(O)CH_3)-CH_2-S-S-CH_2-CH(CH_2Phe)CONHCH_2COO$
$CH_2Phe$.

**7.** Verfahren gemäß einem der Ansprüche 1 bis 4 zur Erhaltung der Verbindung mit der Formel:

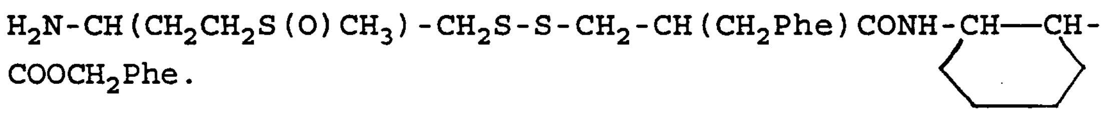

**8.** Verfahren gemäß einem der Ansprüche 1 bis 5 zur Erhaltung der Verbindung mit der Formel:

$H_2N-CH(CH_2CH_2S(O)CH_3)CH_2-S-S-CH_2CH(CH_2CH_2S(O)CH_3)-CONH-$
$CH(CH_3)COOCH_2Phe$.

**9.** Verfahren gemäß einem der Ansprüche 1 bis 5 zur Erhaltung der Verbindung mit der Formel:

$H_2N-CH(CH_2CH_2S(O)CH_3)CH_2-S-S-CH_2-CH(CH_2CH_2S(O)CH_3)-CONH-$
$CH(Phe)-CH_2-COOCH_2Phe$.

**10.** Verfahren gemäß einem der Ansprüche 1 bis 5 zur Erhaltung der Verbindung mit der Formel:

$H_2N-CH(CH_2CH_2S(O)CH_3)CH_2-S-S-CH_2-CH(CH_2CH_2S(O)CH_3)-CONH-$
$CH(CH_2Phe)-CH_2-COOCH_2Phe$.

**11.** Verfahren gemäß einem der Ansprüche 1 bis 5 zur Erhaltung der Verbindung mit der Formel:

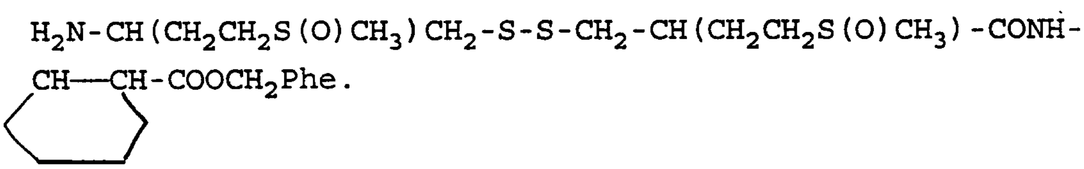

**12.** Verfahren gemäß Anspruch 1 zur Erhaltung der Verbindung mit der Formel:

$H_2N-CH(CH_2CH_2SCH_3)CH_2S-S-CH_2CH(CH_2CH(CH_3)_2)-CONHCH(CH_3)COO$
$CH_2Phe$.

**13.** Verfahren gemäß Anspruch 1 zur Erhaltung der Verbindung mit der Formel:

$H_2N\text{-}CH(CH_2CH_2SCH_3)CH_2S\text{-}S\text{-}CH_2CH(CH_2CH_2SCH_3)\text{-}CONHCH(CH_3)COO$
$CH_2Phe$.

**14.** Verfahren gemäß Anspruch 1 zur Erhaltung der Verbindung mit der Formel:

$H_2N\text{-}CH(CH_2CH(CH_3)_2)\text{-}CH_2\text{-}S\text{-}S\text{-}CH_2\text{-}CH(CH_2Phe)\text{-}CONH\text{-}CH_2COO$
$CH_2Phe$.

**15.** Verfahren gemäß Anspruch 1 zur Erhaltung der Verbindung mit der Formel:

$H_2N\text{-}CH(CH_2CH(CH_3)_2)\text{-}CH_2\text{-}S\text{-}S\text{-}CH_2\text{-}CH(CH_2Phe)\text{-}CONH\text{-}CH(CH_3)COO$
$CH_2Phe$.

**16.** Verfahren gemäß Anspruch 1 zur Erhaltung der Verbindung mit der Formel:

$H_2N\text{-}CH(CH_2CH(CH_3)_2)\text{-}CH_2\text{-}S\text{-}S\text{-}CH_2\text{-}CH(CH_2Phe)\text{-}CONH\text{-}CH(CH_2Phe)$
$COOCH_2Phe$.

**17.** Verfahren gemäß Anspruch 1 zur Erhaltung der Verbindung mit der Formel:

$H_2N\text{-}CH(CH_2CH_2SCH_3)\text{-}CH_2\text{-}S\text{-}S\text{-}CH_2\text{-}CH(CH_2Phe)CONH\text{-}CH_2\text{-}COOCH_2Phe$.

**18.** Verfahren gemäß Anspruch 1 zur Erhaltung der Verbindung mit der Formel:

$H_2N\text{-}CH(CH_2CH_2SCH_3)\text{-}CH_2\text{-}S\text{-}S\text{-}CH_2\text{-}CH(CH_2Phe)CONH\text{-}CH(CH_3)\text{-}COO$
$CH_2Phe$.

**19.** Verfahren gemäß Anspruch 1 zur Erhaltung der Verbindung mit der Formel:

$H_2N\text{-}CH(CH_2CH_2SCH_3)\text{-}CH_2\text{-}S\text{-}S\text{-}CH_2\text{-}CH(CH_2Phe)CONH\text{-}CH(CH_2Phe)$
$COOCH_2Phe$.

**20.** Verfahren gemäß Anspruch 1 zur Erhaltung der Verbindung mit der Formel:

$H_2N\text{-}CH(CH_2CH_2S(O)CH_3)\text{-}CH_2\text{-}S\text{-}S\text{-}CH_2\text{-}CH(CH_2Phe)\text{-}CONH\text{-}$
$CH(CH_2Phe)COOCH_2Phe$.

**21.** Verfahren gemäß Anspruch 1, dadurch gekennzeichnet, daß, wenn $R_2$ eine $CH_3$-Gruppe ist und wenn $R_1$ und $R_4$ gleich sind, die Verbindung mit der Formel (II) erhalten wird, indem man von einer Verbindung ausgeht mit der Formel:

$CH_3\text{-}CO\text{-}SCH(CH_3)CHR_1\text{-}CO_2H$ (XI),

- durch Umsetzung dieser Verbindung zu einem Produkt mit der Formel:

  $CH_3\text{-}CO\text{-}SCH(CH_3)CHR_1\text{-}CON_3$ (XII),

  anschließend zu einem Produkt mit der Formel:

  $CH_3\text{-}CO\text{-}SCH(CH_3)CHR_1\text{-}NHR_8$ (XIII),

- durch anschließende saure Hydrolyse der Acetylthiogruppe.

**22.** Verfahren gemäß Anspruch 1, dadurch gekennzeichnet, daß, wenn $R_3$ ein Wasserstoffatom oder eine $CH_3$-Gruppe ist und wenn AB eine Amidgruppe CONH ist, die Verbindung mit der Formel (V) erhalten wird, indem man von einer Verbindung ausgeht mit der Formel:

$CH_3$-CO-SCH($R_3$)CH($R_4$)-$CO_2$H (XIV),

- durch alkalische Hydrolyse dieser Verbindung, was zur Bildung der Verbindung mit der folgenden Formel führt:

  (SCH($R_3$)CH($R_4$)-$CO_2$H)$_2$ (XV),

- durch anschließende Kondensation mit einem Aminoester der Formel:

  $H_2$N-CH($R_5$)-[CH($R_6$)]$_n$-CO-O$R_7$ (XVI),

  was zur Bildung einer Verbindung mit der folgenden Formel führt:

  (SCH$R_3$CH($R_4$)CONH-CH($R_5$)[CH($R_6$)]$_n$-COO$R_7$)$_2$ (XVII),

- anschließende Behandlung in reduzierendem Milieu.

**23.** Verfahren gemäß Anspruch 1, dadurch gekennzeichnet, daß die Ausgangsverbindung mit der Formel XI des Verfahrens gemaß Anspruch 21 erhalten werden kann, indem man von einer Verbindung ausgeht mit der Formel:

(Phe)$_3$P-CH($R_1$)-COO$CH_3$ (XVIII),

- durch Kondensation mit Acetaldehyd, was zur Bildung der Verbindung mit der folgenden Formel führt:

  $CH_3$CH = C($R_1$)-COO$CH_3$ (XIX),

- durch anschließende alkalische Hydrolyse, was zur Bildung der Verbindung mit der folgenden Formel führt:

  $CH_3$CH = C($R_1$)-$CO_2$H (XX),

- durch anschließendes Zufügen von Thioessigsäure.

**24.** Verfahren gemäß Anspruch 1, dadurch gekennzeichnet, daß, wenn $R_3$ ein Wasserstoffatom oder eine $CH_3$-Gruppe ist und wenn $R_1$ und $R_4$ gleich sind und wenn AB eine umgekehrte Amidgruppe NHCO ist, die Verbindung mit der Formel (V) hergestellt wird, indem man von den Verbindungen (X) bzw. (XIII) ausgeht, denen die folgende allgemeine Formel zukommt:

$CH_3$CO-S-CH($R_2$)CH($R_1$)NH$R_8$ (XXI),

- durch alkalische Hydrolyse, was zur Bildung der Verbindung mit der folgenden Formel führt:

  [SCH($R_2$)CH($R_1$)NH$R_8$]$_2$ (XXII),

- durch anschließende Entfernung der Schutzgruppe von der Aminofunktion, was zur Verbindung mit der folgenden Formel führt:

  [SCH($R_2$)CH($R_1$)$NH_2$]$_2$ (XXIII),

- durch anschließende Kondensation mit Malonsäure- oder Bernsteinsäure-Monoestern mit der Formel:

  $CO_2$H-CH($R_5$)-[CH($R_6$)]$_n$-COO$R_7$ (XXIV),

  was zur Bildung einer Verbindung mit der folgenden Formel führt:

$[SCH(R_2)CHR_1\text{-}NHCO\text{-}CHR_5\text{-}(CHR_6)_n\text{-}COOR_7]_2$,

- durch anschließende Behandlung in reduzierendem Milieu.

**25.** Verfahren zur Herstellung von Verbindungen der Formel I:

$H_2N\text{-}CH(R_1)\text{-}CH(R_2)\text{-}S\text{-}S\text{-}CH(R_3)\text{-}CH(R_4)\text{-}A\text{-}B\text{-}Z$ (I),

in der:
$R_1$ bedeutet:

- eine gesättigte oder ungesättigte, lineare oder verzweigte Alkylkohlenwasserstoffkette mit 1 bis 6 Kohlenstoffatomen, die nicht substituiert oder substituiert ist durch eine OR-Gruppe, in der R ein Wasserstoffatom, eine lineare oder verzweigte $C_1$- bis $C_4$-Kohlenwasserstoffketteoder ein Phenyl- oder Benzylrest ist, eine Thioethergruppe SR, wobei R wie oben definiert ist, oder eine oxidierte Thioethergruppe S(O)R, wobei R wie oben definiert ist;
- eine Methylcycloalkylgruppe mit 5 bis 6 Kohlenstoffatomen, eine Benzyl- oder Phenylgruppe, die gegebenenfalls substituiert sind durch:
  . 1 bis 5 Halogen-, insbesondere Fluoratome,
  . oder eine OR'- oder SR'-Gruppe, wobei R' eine Alkylgruppe mit 1 bis 4 Kohlenstoffatomen ist und der Thioether SR' am Schwefelatom oxidiert oder nicht oxidiert ist,
  . oder eine Aminogruppe, die gegebenenfalls durch eine aliphatische Alkylgruppe mit 1 bis 6 Kohlenstoffatomen mono- oder disubstituiert ist und die an der Aminofunktion oxidiert oder nicht oxidiert ist;
- eine Methylengruppe, die durch einen aromatischen oder gesättigten Heterocyclus mit 5 oder 6 Kohlenstoffatomen substituiert ist, der als Heteroatom ein Stickstoffatom, ein Sauerstoffatom oder ein Schwefelatom besitzt, wobei das Stickstoff- bzw. das Schwefelatom gegebenenfalls in Form eines N-Oxids bzw. S-Oxids oxidiert vorliegen;

$R_2$ bedeutet unabhängig ein Wasserstoffatom oder eine Methylgruppe;
$R_3$ bedeutet ein Wasserstoffatom oder eine lineare oder verzweigte Alkylkette;
$R_4$ bedeutet:

- eine gesättigte oder ungesättigte, lineare oder verzweigte Kohlenwasserstoffkette mit 1 bis 6 Kohlenstoffatomen, die nicht substituiert oder substituiert ist durch eine Hydroxygruppe, eine Ethergruppe OR, in der die Gruppe R das gleiche wie vorher bedeutet, eine Thiolgruppe SH, eine Thioethergruppe SR (wobei R das gleiche wie vorher bedeutet) oder eine zum Sulfoxid oxidierte Thioethergruppe;
- oder eine Methylcycloalkylgruppe mit 5 bis 6 Kohlenstoffatomen, eine Benzyl- oder Phenylgruppe, gegebenenfalls substituiert durch 1 bis 5 Halogen-, insbesondere Fluoratome, eine Hydroxy- oder eine Thiolgruppe, eine Ether- oder Thioethergruppe OR' bzw. SR', wobei R' 1 bis 4 Kohlenstoffatome besitzt und der Thioether am Schwefelatom oxidiert oder nicht oxidiert ist, eine Aminogruppe, die gegebenenfalls durch eine aliphatische Alkylgruppe mit 1 bis 6 Kohlenstoffatomen mono- oder disubstituiert ist und die an der Aminofunktion oxidiert oder nicht oxidiert ist;
- oder eine Methylengruppe, die durch einen aromatischen oder gesättigten Heterocyclus mit 5 oder 6 Kohlenstoffatomen substituiert ist, der als Heteroatom ein Stickstoffatom, ein Sauerstoffatom oder ein Schwefelatom besitzt, wobei das Stickstoff- bzw. das Schwefelatom gegebenenfalls in Form eines N-Oxids bzw. S-Oxids oxidiert vorliegen;

  die Gruppe Z bedeutet:
- eine Morpholingruppe $\text{-}N(C_4H_8O)$
- eine Piperidingruppe $\text{-}N(C_5H_{10})$, die gegebenenfalls durch einen Rest -OH, einen Rest $\text{-}CO_2H$ oder einen Rest $COOR^1$ substituiert ist, wobei $R^1$ eine lineare oder verzweigte Alkylkette mit 1 bis 6 Kohlenstoffatomen, einen Phenyl- oder einen Benzylrest darstellt;
- eine Pyrazolingruppe $\text{-}N(C_4H_8)N\text{-}R^2$, wobei $R^2$ eine Alkylkette mit 1 bis 6 Kohlenstoffatomen, einen Benzylrest oder einen Phenylrest darstellt;

A-B bedeutet eine Amidgruppe CONH oder eine umgekehrte Amidgruppe NHCO;
wobei die Verbindungen in Form racemischer Gemische, in Form von Enantiomeren, Diastereomeren oder Stereoisomeren sowie deren Gemischen sowie ihren pharmazeutisch akzeptablen Salzen vorliegen, und das Verfahren dadurch gekennzeichnet ist, daß Verbindungen mit der Formel

$(SCH(R_3)CH(R_4)\text{-}CO_2H)_2$ (XV)

mit einem Hydrazin der Formel

$H_2N\text{-}Z$ (XXV)

kondensiert werden, was zur Bildung einer Verbindung mit der folgenden Formel führt:

$(SCH(R_3)CH(R_4)\text{-}CONH\text{-}Z)_2$ (XXVI),

welche mit einer Verbindung der Formel

$R_8NHCH(R_1)\text{-}CH(R_2)\text{-}S\text{-}S\text{-}$ 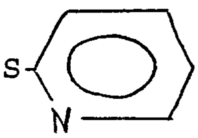 (IV)

kondensiert wird, was zur Verbindung mit der Formel (I) führt.

**26.** Verfahren gemäß Anspruch 25 zur Erhaltung einer Verbindung mit der Formel:

$H_2N\text{-}CH(CH_2CH_2S(O)CH_3)\text{-}CH_2\text{-}S\text{-}S\text{-}CH_2\text{-}CH(CH_2CH(CH_3)_2)\text{-}CONH\text{-}$ 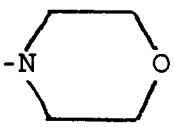

**27.** Verfahren zur Herstellung einer pharmazeutischen Zusammensetzung, dadurch gekennzeichnet, daß man wenigstens eine Verbindung, die nach einem Verfahren gemaß einem der Ansprüche 1 bis 26 erhalten wurde, mit einem oder mehreren verträglichen oder pharmazeutisch akzeptablen Verdünnungsmitteln oder Adjuvantien kombiniert.

**28.** Verfahren gemaß Anspruch 27, dadurch gekennzeichnet, daß die Zusammensetzung zur Behandlung von Depressionen und von Schmerzen bestimmt ist.

**29.** Verfahren gemäß einem der Ansprüche 27 und 28, dadurch gekennzeichnet, daß die Zusammensetzung zur intravenösen, subcutanen, intraperitonealen oder oralen Verabreichung bestimmt ist.